# EUROPEAN PATENT SPECIFICATION

(11) **EP 2 644 199 B1**
(45) Date of publication and mention of the grant of the patent: **11.10.2017**
(21) Application number: 13171200.2
(22) Date of filing: 05.03.2008
(51) Int. Cl.: C12Q 1/68, A61K 31/7088, G01N 33/483, A61K 31/165, A61K 45/06, A61K 48/00, G01N 33/574, C12N 15/113, G01N 33/50

(54) **Methods for detecting and modulating the sensitivity of tumour cells to anti-mitotic agents**
Verfahren zum Nachweis und zur Modulation der Sensitivität von Tumorzellen gegenüber anti-mitotischen Mitteln
Procédés permettant de détecter et de moduler la sensibilité des cellules tumorales à des agents antimitotiques

(30) Priority: 05.03.2007 AU 2007901131 P; 28.09.2007 AU 2007905307 P
(43) Date of publication of application: 02.10.2013
(62) Divisional of application: 08714346.7
(73) Proprietor: NewSouth Innovations Pty Limited, Sydney, NSW 2052 (AU)
(72) Inventor: Kavallaris, Maria, Bondi Beach New South Wales 2026 (AU); Gan, Pei Pei, Randwick New South Wales 2031 (AU)
(74) Representative: Wise, Daniel Joseph

(56) References cited:
- GAN PEI PEI ET AL: "Targeting class III beta-tubulin increases sensitivity to chemotherapeutic agents used in the treatment of non-small cell lung cancer.", PROCEEDINGS OF THE AMERICAN ASSOCIATION FOR CANCER RESEARCH ANNUAL MEETING, vol. 47, April 2006 (2006-04), page 555, XP002711603, & 97TH ANNUAL MEETING OF THE AMERICAN-ASSOCIATION-FOR-CANCER-RESEARCH (AACR); WASHINGTON, DC, USA; APRIL 01 -05, 2006 ISSN: 0197-016X
- RANGANATHAN S ET AL: "INCREASE OF BETAIII- AND BETAIVA-TUBULIN ISOTYPES IN HUMAN PROSTATE CARCINOMA CELLS AS A RESULT OF ESTRAMUSTINE RESISTANCE", CANCER RESEARCH, AMERICAN ASSOCIATION FOR CANCER RESEARCH, US, vol. 56, no. 11, 1 June 1996 (1996-06-01), pages 2584-2589, XP001084220, ISSN: 0008-5472
- KAVALLARIS M ET AL: "ANTISENSE OLIGONUCLEOTIDES TO CLASS 3 BETA-TUBULIN SENSITIZE DRUG-RESISTANT CELLS TO TAXOL", BRITISH JOURNAL OF CANCER, NATURE PUBLISHING GROUP, LONDON, GB LNKD- DOI:10.1038/SJ.BJC.6690507, vol. 80, no. 7, 1 June 1999 (1999-06-01), pages 1020-1025, XP000964803, ISSN: 0007-0920
- PARADISO A ET AL: "Biomarkers predictive for clinical efficacy of taxol-based chemotherapy in advanced breast cancer.", ANNALS OF ONCOLOGY : OFFICIAL JOURNAL OF THE EUROPEAN SOCIETY FOR MEDICAL ONCOLOGY / ESMO MAY 2005 LNKD- PUBMED:15923415, vol. 16 Suppl 4, May 2005 (2005-05), pages IV14-IV19, XP002589956, ISSN: 1569-8041
- DRUKMAN SIMA ET AL: "Microtubule alterations and resistance to tubulin-binding agents (review)", INTERNATIONAL JOURNAL OF ONCOLOGY, DEMETRIOS A. SPANDIDOS ED. & PUB, GR, vol. 21, no. 3, 1 January 2002 (2002-01-01), pages 621-628, XP008123826, ISSN: 1019-6439
- P. P. GAN ET AL: "Class III -Tubulin Mediates Sensitivity to Chemotherapeutic Drugs in Non Small Cell Lung Cancer", CANCER RESEARCH, vol. 67, no. 19, 1 October 2007 (2007-10-01), pages 9356-9363, XP55075906, ISSN: 0008-5472, DOI: 10.1158/0008-5472.CAN-07-0509
- KAHLIN CHEUNG-ONG ET AL: "DNA-Damaging Agents in Cancer Chemotherapy: Serendipity and Chemical Biology", CHEMISTRY & BIOLOGY, vol. 20, no. 5, 1 May 2013 (2013-05-01), pages 648-659, XP55141833, ISSN: 1074-5521, DOI: 10.1016/j.chembiol.2013.04.007
- CHOLLET PATRICE ET AL: "Side-effects of a systemic injection of linear polyethylenimine-DNA complexes.", THE JOURNAL OF GENE MEDICINE 2002 JAN-FEB, vol. 4, no. 1, January 2002 (2002-01), pages 84-91, ISSN: 1099-498X

## Description

### Related Applications

This application claims priority from Australian provisional patent application No 2007901131, filed 5 March 2007, and from Australian provisional patent application No. 2007905307, filed 28 September 2007.

### Technical Field

Methods for screening for tumour sensitivity to anti-mitotic agents based on the characterisation of β-tubulin expression by the tumour, methods for modulating the expression of β-tubulin, and in particular II-, III-, or IVb- β-tubulin, in tumour cells are disclosed. Molecules and methods for enhancing the sensitivity of tumour cells to tubulin-binding agents, such as vinca alkaloids, taxanes and epothilones and to other anti-cancer agents are also disclosed. The invention relates to nucleic acid constructs and to siRNA molecules comprising a nucleotide sequence specific to at least a portion of the class III β-tubulin gene product for use in increasing the sensitivity of a tumour cell *in vivo* to a DNA-damaging agent selected from a platinum-based DNA damaging agent, cisplatin, doxorubicin or etoposide, wherein the expression of the construct, or the siRNA molecule, is able to decrease the expression of class III β-tubulin in the tumour cell to thereby increase sensitivity of the tumour cell to the DNA damaging agent, and wherein polyethylenimine (PEI) is present as a delivery vehicle.

The invention also relates to a pharmaceutical composition comprising (i) a nucleic acid construct comprising a nucleotide sequence encoding a short hairpin RNA (shRNA) or an siRNA sequence specific to at least a portion of a class III β-tubulin gene, and a pharmaceutically acceptable carrier, diluent or excipient, wherein expression of the construct is able to decrease the expression of class III β-tubulin in the tumour, and wherein polyethylenimine (PEI) is present as a delivery vehicle or (ii) a siRNA molecule comprising a sequence specific to at least a portion of a class III β tubulin gene, and a pharmaceutically acceptable carrier, diluent or excipient, wherein introduction of the siRNA molecule to the tumour is able to decrease the expression of class III β-tubulin in the tumour, and wherein polyethylenimine (PEI) is present as a delivery vehicle. The invention also relates to a nucleic acid construct comprising a nucleotide sequence specific to at least a portion of a class III β-tubulin gene product, for use in treating a tumour cell in a subject, wherein the nucleic acid construct decreases the expression of class III β-tubulin in the tumour cell and thereby increases the sensitivity of the tumour cell to a tubulin-binding agent, and at least one tubulin-binding agent is administered to the subject, and wherein polyethylenimine (PEI) is present as a delivery vehicle for the nucleic acid construct.

### Background

Microtubules are required for cell division in eukaryotic cells, functioning as part of the spindle to ensure chromosome separation and segregation. The principal components of microtubules are α- and β-tubulin, which form heterodimers. At least seven different β-tubulin isotypes have been identified in humans to date. These isotypes are classified according to the sequence of carboxy terminal domains as follows (with the Roman numeral class numbers referring to the protein isotype and the human gene classification in parentheses): class I (HM40), class II (Hβ9), class III (Hβ4), class IVa (H5β), class IVb (Hβ2), class V and class VI (Hβ1). Different isotypes exhibit distinct patterns of tissue expression. For example, class III β-tubulin is normally expressed only in neuronal cells.

Tubulin-binding agents are an important component in the treatment of many human cancers, including ovarian cancer, breast cancer, non small cell lung cancer, prostate cancer, advanced stage neuroblastoma and various lymphomas. These clinically important agents include taxanes, such as paclitaxel (Taxol), epothilones, and vinca alkaloids such as vinblastine, which bind to β-tubulin from α/β-tubulin and disrupt microtubule dynamics and thereby induce mitotic arrest and apoptosis.

Upon binding to tubulin, taxanes and epothilones are known to enhance microtubule assembly, resulting in the stabilization of polymerized microtubules, abnormal spindle esters and cell cycle arrest in mitosis. In contrast, vinca alkaloids are a second class of tubulin-binding agents which induce the destabilization of polymerized tubulin by blocking the region involved in tubulin dimer attachment, thereby preventing microtubule assembly. Platinum-based DNA-damaging agents are a large class of anti-cancer drugs which bind and modify DNA, which are effective in the treatment of a variety of different cancers, and which are commonly administered in combination therapies with tubulin-binding agents.

The development of resistance by tumour cells to microtubule-binding agents such as taxanes, epothilones and vinca alkaloids is, however, a significant obstacle to the success of a range of chemotherapeutic regimens.

Lung cancer is the most common cancer in the world with over one million cases diagnosed every year and remains the leading cause of cancer death in both men and women. Advanced non-small cell lung carcinoma (NSCLC) accounts for more than 80% of these cases. More than half of these subjects have developed metastasis at the time of diagnosis, and so chemotherapy remains the most effective treatment option. Over the last decade, the use in phase 2 studies of chemotherapeutic agents such as paclitaxel and vinorelbine, either as a single agent or as part of a combination chemotherapeutic regimen, has shown promising activity and prolonged survival rates at one year. However, in general the prognosis for subjects remains poor due to the emergence of drug-resistant tumour cells that significantly limits the clinical utility of these drugs in the treatment of lung cancer. Accordingly, there is a clear need for methods and approaches to combat and overcome this resistance to ensure the continued efficacy of anti-mitotic agents in the treatment of cancer.

On the basis of differential tissue expression and highly conserved sequences across all species, it has been suggested that each β-tubulin isotype may provide unique characteristics which impart functional differences in microtubules. Although alterations in tubulin isotype expression have been demonstrated in cell lines selected for resistance to antimicrotubule agents, including paclitaxel, docetaxel and estramustine, the available data focuses on the role of βIII tubulin in paclitaxel resistance. The relevance of βIII tubulin in the response of cells to other types of tubulin-binding agents, or the relevance of non-βIII-tubulin isotypes in responses to tubulin-binding agents is poorly understood. Class II β-tubulin, for example, was found in both normal and tumour breast tissues, suggesting that it may not be a good biomarker for these tumours. Further, despite the common use of DNA-damaging agents in combination therapy with *vinca* alkaloids, the effects of βIII-tubulin expression on the efficacy of these agents has not been addressed.

One particular difficulty with the studies of drug resistant cells is to ascertain whether changes in β-tubulin expression which may be observed in resistant cells contributes to the resistant phenotype or arise as a secondary mechanism. It is not unusual for multidrug resistant cells to undergo multiple cellular changes and they hence may incorporate more than one mechanism of resistance.

In view of the above, for therapeutic applications, there is a need for agents which enhance tumour sensitivity to tubulin-binding agents and/or other anti-mitotic agents or DNA -damaging agents. In addition, it would be desirable to be able to screen a tumour to predict its sensitivity or potential sensitivity to a range of tubulin-binding agents and/or other anti-mitotic agents, both before commencing treatment with such agents and during the course of treatment.

Gan and Kavallaris, 2006, Proc AACR, 47, 555 is a conference abstract disclosing *in vitro* data on the effects of downregulating βIII tubulin expression in two lunch cancer cell lines. Kavallaris et al 1999, British Journal of Cancer 80(7), 1020-1025 discloses antisense oligonucleotides to class IIIβ-tubulin sensitise drug resistant cells to Taxol.

### Summary

The present inventors have demonstrated an association between the expression of class II, class III or class IVb β-tubulin in NSCLC cells and leukemic cells with the response of these cells to various tubulin-binding agents.

The invention provides a nucleic acid construct comprising a nucleotide sequence specific to at least a portion of the class III β-tubulin gene product for use in increasing the sensitivity of a tumour cell *in vivo* to a DNA-damaging agent selected from a platinum-based DNA damaging agent, cisplatin, doxorubicin or etoposide, wherein expression of the construct is able to decrease the expression of class III β-tubulin in the tumour cell to thereby increase sensitivity of the tumour cell to the DNA damaging agent, and wherein polyethylenimine (PEI) is present as a delivery vehicle.

The invention also provides a pharmaceutical composition for use in increasing the sensitivity of a tumour to a DNA-damaging agent selected from a platinum-based DNA damaging agent, cisplatin, doxorubicin or etoposide, the pharmaceutical composition comprising a nucleic acid construct comprising a nucleotide sequence encoding a short hairpin RNA (shRNA) or an siRNA sequence specific to at least a portion of a class III β-tubulin gene, and a pharmaceutically acceptable carrier, diluent or excipient, wherein expression of the construct is able to decrease the expression of class III β-tubulin in the tumour, and wherein polyethylenimine (PEI) is present as a delivery vehicle.

The invention also provides a siRNA molecule comprising a nucleotide sequence specific to at least a portion of the class III β-tubulin gene product for use in increasing the sensitivity of a tumour cell *in vivo* to a DNA-damaging agent selected from a platinum-based DNA damaging agent, cisplatin, doxorubicin or etoposide, wherein the siRNA molecule is able to decrease the expression of class III β-tubulin in the tumour cell to thereby increase sensitivity of the tumour cell to the DNA-damaging agent, and wherein polyethylenimine (PEI) is present as a delivery vehicle.

The invention also provides a pharmaceutical composition for use in increasing the sensitivity of a tumour a DNA-damaging agent selected from a platinum-based DNA damaging agent, cisplatin, doxorubicin or etoposide, the pharmaceutical composition comprising a siRNA molecule comprising a sequence specific to at least a portion of a class III β -tubulin gene, and a pharmaceutically acceptable carrier, diluent or excipient, wherein introduction of the siRNA molecule to the tumour is able to decrease the expression of class III β -tubulin in the tumour, and wherein polyethylenimine (PEI) is present as a delivery vehicle.

The invention also provides a nucleic acid construct comprising a nucleotide sequence specific to at least a portion of a class III β-tubulin gene product, for use in treating a tumour cell in a subject, wherein the nucleic acid construct decreases the expression of class III β-tubulin in the tumour cell and thereby increases the sensitivity of the tumour cell to a tubulin-binding agent, and at least one tubulin-binding agent is administered to the subject, and wherein polyethylenimine (PEI) is present as a delivery vehicle for the nucleic acid construct.

A method of screening a tumour cell for resistance to or potential resistance to a tubulin-binding agent, the method comprising detecting the expression of any one or more of class II, class III and class IVb β-tubulin by the tumour cell, wherein the expression of any one or more of class II, class III and class IVb β-tubulin indicates that the tumour cell has resistance or potential resistance to the tubulin-binding agent is disclosed.

In one embodiment, the tubulin-binding agent is a microtubule destabilizing agent. In one embodiment, the tubulin-binding agent is a microtubule stabilizing agent.

In one embodiment the method comprises detecting the expression of any two of class II, class III and class IVb β-tubulin by the tumour cell. In another embodiment, the method comprises detecting the expression of class II, class III and class IVb β-tubulin by the tumour cell.

In certain embodiments, the step of detecting the expression of one or more of class II, class III and class IVb β-tubulin by the tumour cell comprises detecting the expression of one or more of class II, class III and class IVb β-tubulin protein.

In certain embodiments the step of detecting the expression of one or more of class II, class III and class IVb β-tubulin by the tumour cell comprises detecting the expression of one or more of class II, class III and class IVb β-tubulin mRNA.

The step of detecting the expression of one or more of class II, class III and class IVb β-tubulin by the tumour cell may comprise quantifying the level of expression of the tubulin.

The step of detecting the expression of one or more of class II, class III and class IVb β-tubulin by the tumour cell may comprise comparing the expression of one or more of class II, class III and class IVb β-tubulin by the tumour cell with the expression of one or more of class II, class III and class IVb β-tubulin by a control cell. The control cell may be tumour cell or a non-tumour cell, for example a cell taken from tissue surrounding the tumour. The control cell may be a tumour cell which is resistant to the tubulin-binding agent. The control cell may be a tumour cell which is sensitive to the tubulin-binding agent. The control cell may be a tumour cell in which the expression of any one or more of class II, class III and class IVb β-tubulin has been reduced.

Also disclosed is a method of assessing the resistance of a tumour cell to a tubulin-binding agent. The method includes detecting an amount of a class II, class III and class IVb β-tubulin protein or a class II, class III and class IVb β-tubulin mRNA by the tumour cell. A subject tumour cell tubulin amount is thereby obtained. The subject tumour cell tubulin amount is compared to a control cell tubulin amount thereby determining a tubulin amount difference. The control cell tubulin amount is a respective amount of a class II, class III or class IVb β-tubulin protein or any one or more of class II, class III and class IVb β-tubulin mRNA in a control cell. The resistance of the tumour cell to the tubulin-binding agent is assessed based on the tubulin amount difference.

The control cell of the above methods may be a control non tumour cell. The control cell of the above methods may be a control tumour cell. The control tumour cell may be a tumour cell which is resistant to the tubulin-binding agent, or a tumour cell which is sensitive to the tubulin-binding agent.

In certain embodiments the detecting of an amount of a class II, class III and class IVb β-tubulin protein is detecting an amount of class II, class III and class IVb β-tubulin protein. In a particular embodiment, the detecting of an amount of any one or more of class II, class III and class IVb β-tubulin protein is detecting an amount of class III β-tubulin protein only.

In other embodiments, the detecting of an amount of a class II, class III and class IVb β-tubulin mRNA is detecting an amount of class II, class III and class IVb β-tubulin mRNA. In a particular embodiment the detecting of an amount of any one or more of class II, class III and class IVb β-tubulin mRNA is detecting an amount of class III β-tubulin mRNA only. Thus, where "a" class II, class III or class IVb β-tubulin protein or mRNA is detected, any one or more of the class II, class III or class IVb β-tubulin protein or mRNA is detected.

A method for enhancing the sensitivity of a tumour cell to at least one tubulin-binding agent, the method comprising introducing into the tumour cell an effective amount of at least one nucleic acid construct comprising a nucleotide sequence specific to at least a portion of the class II, class III or class IVb β-tubulin gene product, wherein the construct decreases the expression of class II, class III or class IVb β-tubulin in the tumour cell is disclosed.

A method for treating a tumour in a subject, comprising administering to the subject an effective amount of at least one nucleic acid construct comprising a nucleotide sequence specific to at least a portion of the class II, class III or class IVb β-tubulin gene product and administering to the subject at least one tubulin-binding agent, wherein the nucleic acid construct decreases the expression of class II, class III or class IVb β-tubulin in the tumour and thereby increases the sensitivity of the tumour to the at least one tubulin-binding agent is also disclosed.

In one embodiment, the nucleic acid construct and the at least one tubulin-binding agent are administered simultaneously. In one embodiment, the nucleic acid construct and the at least one tubulin-binding agent are administered concurrently. In another embodiment, the nucleic acid construct is administered before the at least one tubulin-binding agent.

In one embodiment of the above disclosures, the nucleotide sequence is an antisense sequence. In another embodiment, the nucleotide sequence is an siRNA sequence. In another embodiment, the nucleotide sequence is a short hairpin RNA. In yet another embodiment, the nucleotide sequence is a ribozyme sequence.

In a particular embodiment of any of the above disclosures, the nucleotide sequence is specific to at least a portion of the class IVb β-tubulin gene product. In another particular embodiment, the nucleotide sequence is specific to at least a portion of the class II β-tubulin gene product. In a particular embodiment, the nucleotide sequence is specific to at least a portion of the class III β-tubulin gene product.

In a particular embodiment of any of the above disclosures, the at least one tubulin-binding agent may be at least one microtubule destabilizing agent. The at least one microtubule destabilizing agent may be selected from the group consisting of any one or more of vinca alkaloid agents, dolostatins, colchicines, cryptophycins, curacin A, 2-methoxyestradiol and derivatives, analogues or prodrugs thereof. In a particular embodiment, the microtubule destabilizing agent is a vinca alkaloid agent. In one embodiment, the vinca alkaloid agent is selected from the group consisting of any one or more of vincristine, vinblastine, vinflunine, vindesine, and vinorelbine, or a derivative, analogue or prodrug thereof.

In a particular embodiment of any of the above disclosures, the at least one tubulin-binding agent may be at least one microtubule stabilizing agent. The at least one microtubule stabilizing agent may be selected from the group consisting of any one or more of a taxane and an epothilone. The taxane may be paclitaxel or docetaxel. The epothilone may be epothilone A, epothilone B or epothilone D. In a particular embodiment of the above aspects, the tubulin-binding agent is a vinca alkaloid agents, a dolostatin, colchicine, a cryptophycin, curacin A, 2-methoxyestradiol or an epothilone.

In a particular embodiment, the tumour cells are non-small cell lung carcinoma cells (NSCLCs).

In a particular embodiment, the subject is a human.

The use of at least one nucleic acid construct comprising a nucleotide sequence specific to at least a portion of the class II, class III or class IVb β-tubulin gene, wherein the construct is able to decrease the expression of class II, class III or class IVb β-tubulin in a tumour cell, in the preparation of a medicament for increasing the sensitivity of a tumour cell to at least one tubulin-binding agent is also disclosed.

Also disclosed is a pharmaceutical composition for increasing the sensitivity of a tumour to at least one tubulin-binding agent, the pharmaceutical composition comprising at least one nucleic acid construct comprising a nucleotide sequence specific to at least a portion of the class II, class III or class IVb β-tubulin gene, wherein the construct is able to decrease the expression of class II, class III or class IVb β-tubulin in a tumour, together with a pharmaceutically acceptable carrier, diluent or excipient.

A kit when used for assessing the sensitivity of a tumour to a tubulin-binding agent, the kit comprising at least one nucleic acid construct comprising a nucleotide sequence specific to at least a portion of the class II, class III or class IVb β-tubulin gene product, wherein the construct is used to detect the expression of class II, class III or class IVb β-tubulin in the tumour is also disclosed. In certain embodiments, the kit further comprises one or more tubulin-binding agents. In particular embodiments, the diagnosing the sensitivity of a tumour to a tubulin-binding agent is an *in vitro* diagnosis. A kit when used for assessing the sensitivity of a tumour to a tubulin-binding agent, the kit comprising at least one antibody which is specific to any one of class II, class III or class IV β-tubulin polypeptide, wherein the antibody is used to detect the expression of class II, class III or class IV β-tubulin in the tumour is disclosed.

A kit when used for treating a tumour, the kit comprising (i) a pharmaceutical composition comprising at least one nucleic acid construct comprising a nucleotide sequence specific to at least a portion of the class II, class III or class IVb β-tubulin gene, together with a pharmaceutically acceptable carrier, diluent or excipient, and optionally (ii) one or more tubulin-binding agents is also disclosed

A veterinary composition for increasing the sensitivity of a tumour to one or more tubulin-binding agents, the veterinary composition comprising at least one nucleic acid construct comprising a nucleotide sequence specific to at least a portion of the class II, class III or class IVb β-tubulin gene, together with a acceptable carrier, diluent or excipient is disclosed.

A method of screening for agents which disrupt microtubule dynamics associated with class II, class III and/or class IVb β-tubulin, the method comprising exposing a candidate agent which is suspected of disrupting microtubule dynamics associated with at least one of class II, class III or class IVb β-tubulin to a first cell which expresses at least one of class II, class III or class IVb β-tubulin and detecting the response of the cell to the candidate agent, exposing the candidate agent to a second cell of the same type in which the expression of at least one of class II, class III or class IVb β-tubulin in the cell has been decreased and detecting the response of the second cell to the candidate agent, and comparing the responses of the first and second cells to the candidate agent, wherein an altered response of the second cell to the candidate agent compared to the response of the first cell signifies an activity for the candidate agent in disrupting microtubule dynamics associated with class II, class III or class IVb β-tubulin is disclosed. In particular embodiments the response of the cell is an increase or decrease in the rate of apoptosis. In particular embodiments the response of the cell is a change in the rate of cell division.

### Abbreviations

- β₂M: beta-2 microglobulin
- FITC: Fluorescein isothiocyanate
- GAPDH: Glyceraldehyde 3-phosphate dehydrogenase
- NSCLC: non-small cell lung carcinoma
- shRNA: short hairpin RNA
- siRNA: short interfering RNA

### Definitions

In the context of this specification, the term "specific" when used in relation to the nucleotide sequence of an antisense, ribozyme, shRNA or siRNA construct means substantially specific, but not necessarily exclusively so. That is, while being specific for class II, class III or class IVb β-tubulin gene product, the nucleotide sequence may also cross-hybridise with other β-tubulin isotype gene product sequences in an amount sufficient to also inhibit their expression. It may be preferable that the nucleotide sequence does not decrease the expression of non-class II, class III and/or class IVb β-tubulin isotypes, or that it decreases the expression of these other isotypes by a lesser proportion than it decreases the expression of class II, class III and/or class IVb isotypes. Further, for example, the nucleotide sequence of an siRNA construct may display less than 100% sequence identity with the class II, class III or class IVb β-tubulin gene and yet retain specificity thereto.

The term "specific" when used in relation to an antibody which is specific to class II, class III or class IVb β-tubulin polypeptide is intended to encompass an antibody which is capable of distinguishing one of these tubulin polypeptide isotypes from other tubulin polypeptide isotypes, for example in an ELISA or Western blot assay. In the case of an antibody which is "specific" for class IVb β-tubulin polypeptide, the antibody will recognise polypeptides of the class IV β-tubulin isotype, while not recognising class II or class III β-tubulin isotypes. An antibody specific for class IVb β-tubulin isotype need not necessarily be able to distinguish between the class IVa and class IVb β-tubulin polypeptides.

As used herein the term "effective amount" includes within its meaning a sufficient amount of an agent or compound to provide the desired therapeutic or prophylactic effect. The exact amount required will vary from subject to subject depending on factors such as the species being treated, the age and general condition of the subject, the severity of the condition being treated, the particular agent being administered and the mode of administration and so forth. Thus, it is not possible to specify an exact "effective amount". However, for any given case, an appropriate "effective amount" may be determined by one of ordinary skill in the art using only routine experimentation. Preferably the effective amount will be substantially non-toxic to the subject while being toxic to the tumour.

As used herein, a construct which "decreases the expression" of class II, class III and/or IVb β-tubulin in a cell is intended to encompass not only the complete inhibition of the expression of any one, any two or all three of these genes, but also diminution of tubulin gene product or polypeptide expression which is sufficient to enhance the sensitivity of the cell to one or more tubulin-binding agents. Thus a decrease in expression of class II, class III and/or IVb β-tubulin mRNA or polypeptide in a cell of at least 10%, at least 20%, at least 30%, at least 40%, at least 50%, at least 60%, at least 70%, at least 80% or at least 90% when compared with a cell not exposed to the construct is contemplated. It may be preferable that the decrease in expression of the class II, class III and/or IVb β-tubulin in a cell is sufficient to obtain a maximal enhancement of sensitivity of the cell to one or more microtubule desensitizing agents. It will be understood that the decrease of expression of class II, class III and/or IVb β-tubulin by the construct may be permanent, for example where the construct is stably incorporated into the genome of the somatic cell, such as the tumour cell, to which it is exposed, or may be only temporary.

In the context of this specification, the term "comprising" means "including, but not necessarily solely including". Furthermore, variations of the word "comprising" such as "comprise" and "comprises" have correspondingly varied meanings.

Throughout this specification, reference to "a" or "one" element does not exclude the plural, unless context determines otherwise. For instance, reference to "a nucleic acid construct" should not be read as excluding the possibility of multiple copies of such nucleic acid constructs. Similarly, reference throughout the specification to "a tumour cell" is intended to be read as including either a single tumour cell or a plurality of tumour cells, for example a collection of cells obtained from a tumour.

The term "any one or more of class II, class III and class IVb β-tubulin" is intended to encompass any one of class II β-tubulin, class III β-tubulin, class IVb β-tubulin, class II and class III β-tubulin, class II and class IVbβ-tubulin, class III and class IVb β-tubulin, and class II, class III and class IVb β-tubulin.

The term "sensitivity" and corresponding terms such as "sensitive" in the context of the interaction between a tumour cell and a tubulin-binding agent or DNA damaging agent is intended to encompass the response of the tumour cell to the anti-mitotic and/or cytotoxic activities of the tubulin-binding agent or DNA damaging agent. Thus, tumour cells with an enhanced sensitivity to a tubulin-binding agent may demonstrate a greater degree of inhibition of mitosis, and/or may exhibit a greater degree of cell death on exposure to a tubulin-binding agent than tumour cells which are less sensitive.

Alternatively, or in addition, a tumour cell which has an increased or enhanced sensitivity to a tubulin-binding agent may respond to the anti-mitotic and/or cytotoxic activity of the tubulin-binding agent where previously it did not respond.

In addition or alternatively, tumour cells with an increased or enhanced sensitivity to a tubulin-binding agent may exhibit an enhanced sensitivity to ionizing radiation and/or a DNA damaging agent on exposure to the tubulin-binding agent.

Tumour cells with an increased or enhanced sensitivity to a tubulin-binding agent may also respond to the anti-mitotic and/or cytotoxic activities of a tubulin-binding agent at lower concentrations of the tubulin-binding agent than a cell which is less sensitive.

In the context of tumour cells *in vitro or in situ*, an enhanced sensitivity to one or more tubulin-binding agents may be expressed in terms of an enhanced decrease in the number of viable cells in culture or *in situ*, an enhanced decrease in the progression of tumour enlargement or an enhanced reduction in the size of the tumour on treatment with the one or more tubulin-binding agents. Conversely, the term "resistant" and similar terms such as "resistance" have a corresponding but opposite meaning to "sensitive".

The resistance of tumour cells to a tubulin-binding agent or a DNA-damaging agent may be directly assessed by a variety of methods available in the art. Examples of such techniques include exposing tumour cells to a series of dilutions of the tubulin-binding agent or DNA damaging agent and determining the concentration at which 50% killing is observed, or the concentration at which the rate of apoptosis is increased or inhibition of clonogenic growth of the cells or the concentration at which the rate of cell division is decreased. Suitable assays are described, for example, in Verrills et al. (2003). Chemistry and Biology 10: 597-607, and in Gan et al. (2007) Cancer Research 67:(19) 9356-9363..

Similarly, where the "response" of a tumour cell to a tubulin-binding agent is described, it is contemplated that the response may include any one or both of the presence of tumour cell apoptosis or a reduction or halting of the rate of tumour cell division.

The term "tubulin-binding binding agent" is intended to include molecules which modulate the dynamics of tubulin polymerisation and/or depolymerization within a cell, and comprises microtubule destabilizing agents and microtubule stabilizing agents.

The term "microtubule destabilizing agent" is intended to broadly encompass the class of compounds which bind to tubulin dimers and which destabilize the formation of the tubulin dimers into microtubules. Tubulin and microtubules are commonly targeted for chemotherapy, as their functions are often dysregulated in many types of cancer. Agents which target tubulin or microtubules constitute one of the most effective classes of chemotherapeutics amongst anticancer agents presently in use for the prolongation of survival of subjects with advanced disease. Typically, microtubule destabilizing agents may bind the vinca domain or the colchicine domain of tubulin. A variety of antimitotic agents which interact with tubulin including a variety of microtubule destabilizing agents are described in Hamel (1996) Med. Res. Rev. 16:207-231. Microtubule destabilizing agents may be vinca alkaloid agents, dolostatins, colchicines, cryptophycins, curacin A, 2-methoxyestradiol or derivatives, analogues or prodrugs thereof. The term microtubule destabilizing agent is not intended to encompass compounds which bind to the microtubule polymer and stabilize microtubules, such as the taxanes (which comprises paclitaxel and docetaxel) and the epothilones.

The term "vinca alkaloid agent" is intended to broadly encompass the class of alkaloid compounds which were originally derived from plants of the genus *Catharanthus*, which possess antimicrotubule activity and which act as mitotic inhibitors. Members of the class include, but are not limited to, vinblastine, vincristine, vinflunine, vindesine, and vinorelbine, and semisynthetic or synthetic analogues or derivatives thereof.

The term "microtubule stabilizing agent" is intended to encompass those compounds which bind to polymerised microtubules and which inhibit the depolymerization of the polymerized microtubules. Compounds which are microtubule stabilizing agents comprise, but are not limited to, the taxanes and the epothilones.

### Brief Description of the Figures

One or more preferred embodiments of the present invention will now be described, by way of an example only, with reference to the accompanying figures, wherein:

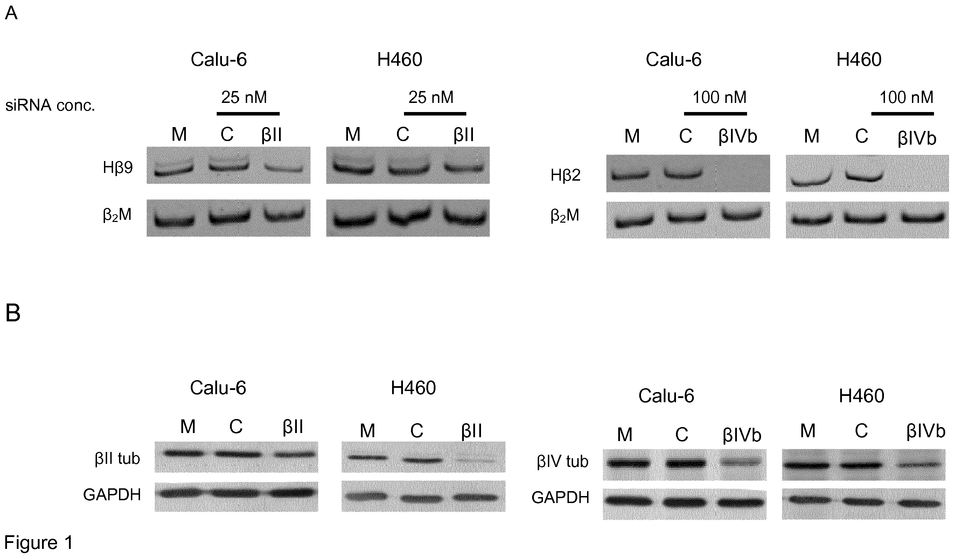

Figure 1 illustrates that siRNA directed against βII or βIVb-tubulin specifically inhibits its respective expression. Figure 1A demonstrates gene product (mRNA) expression of βII (Hβ9) and βIVb (Hβ2) in two NSCLC cell lines, Calu-6 and H460, as determined by semi-quantitative reverse transcription-PCR polyacrylamide gels, at 48h after transfection with siRNA directed against βII and βIVb at 25 nM and 100 nM respectively. The housekeeping gene beta-2 microglobulin (β₂M) which is expressed at relatively constant levels was used to normalise the levels of expression of each of the tubulin mRNAs. Figure 1B demonstrates the expression of βII- and βIVb-tubulin protein 72h after transfection identified by western blots. GAPDH (Glyceraldehyde 3-phosphate dehydrogenase) polypeptide expression was detected simultaneously as the loading control. M: mock transfection; C: control siRNA-transfection; βII: class II β-tubulin siRNA-transfection; βIVb: class IVb β-tubulin siRNA-transfection; β₂M: β₂-microglobulin control.

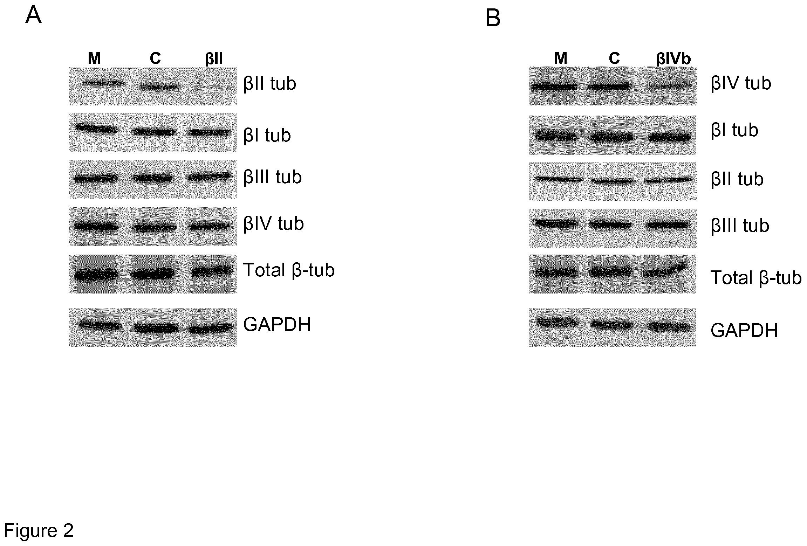

Figure 2 illustrates the specificity of the siRNAs which were used, as demonstrated in western blots. Figure 2A demonstrates βII siRNA specificity. Figure 2B demonstrates βIVb siRNA specificity. No significant changes in the level of protein expression were observed for other β-tubulin isotypes in βII or βIVb H460 knockdown cells.

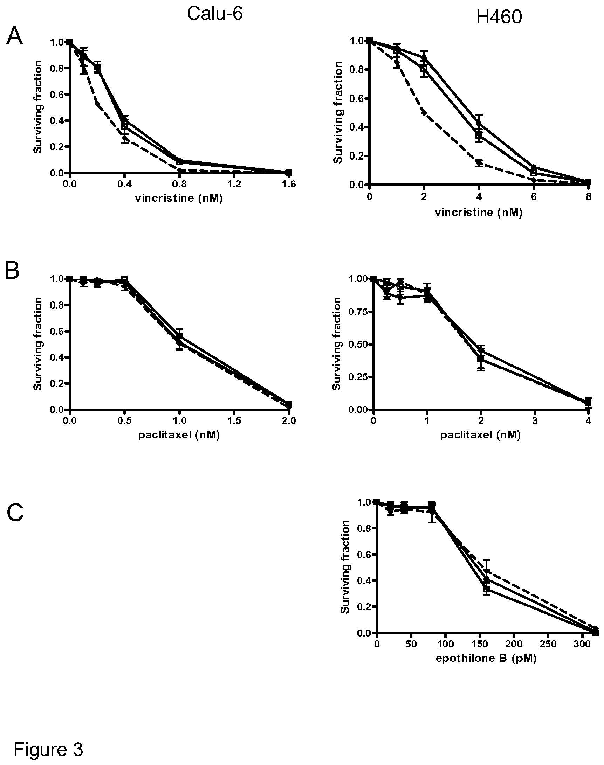

Figure 3 illustrates the effect of class II β-tubulin knockdown on sensitivity of two NSCLC cell lines, Calu-6 and H460, to tubulin-binding agents. Clonogenic assays were performed on mock-transfected cells (closed circles, solid line), control siRNA-transfected cells (open squares, solid line) and βII siRNA-transfected cells (closed diamonds, broken line). The graphs show the clonogenic survival of cells exposed to A vincristine, B paclitaxel and C epothilone, expressed as surviving fraction. Data represents mean ± SEM of at least four independent experiments.

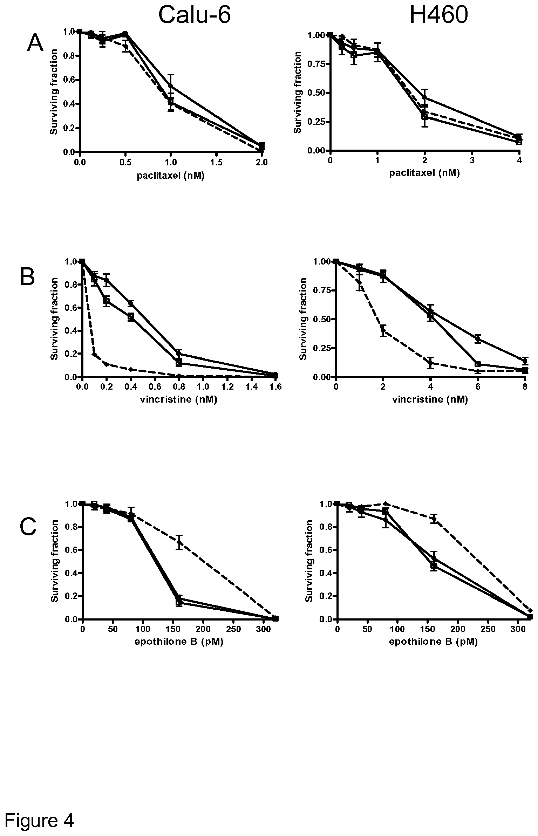

Figure 4 illustrates the effect of class βIVb-tubulin knockdown on the sensitivity of two NSCLC cell lines, Calu-6 and H460, to tubulin-binding agents. Clonogenic assays were performed on mock-transfected cells (closed circles, solid line), control siRNA-transfected cells (open squares, solid lines) and βIVb siRNA-transfected cells (closed diamond, broken line). The graphs show the clonogenic survival of cells exposed to A vincristine, B paclitaxel and C the microtubule stabilizing agent epothilone B, expressed as surviving fraction. Data represents mean ± SEM of at least four independent experiments.

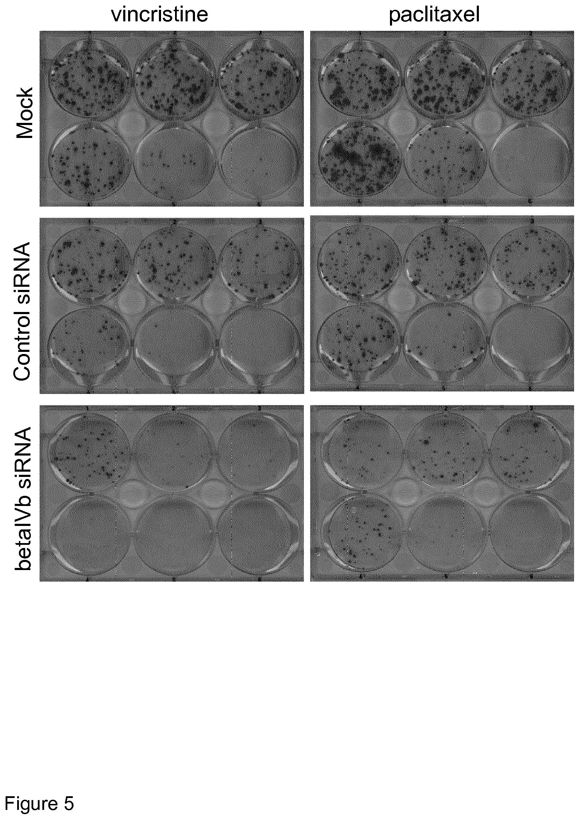

Figure 5 provides photographs of cultured colonies of the tumour cell line Calu-6 which illustrates the dose response of Calu-6 transfected cells to vincristine (left hand column) and paclitaxel (right hand column). After 24h transfection, approximately 600 cells of each transfected cell population were seeded into 6-well plates containing increasing concentrations of tubulin-binding agent. Plates were stained with crystal violet when visible colonies formed. Cells transfected with βIVb siRNA were rendered hypersensitive to vincristine, but transfection had no effect on sensitivity to paclitaxel when compared to controls.

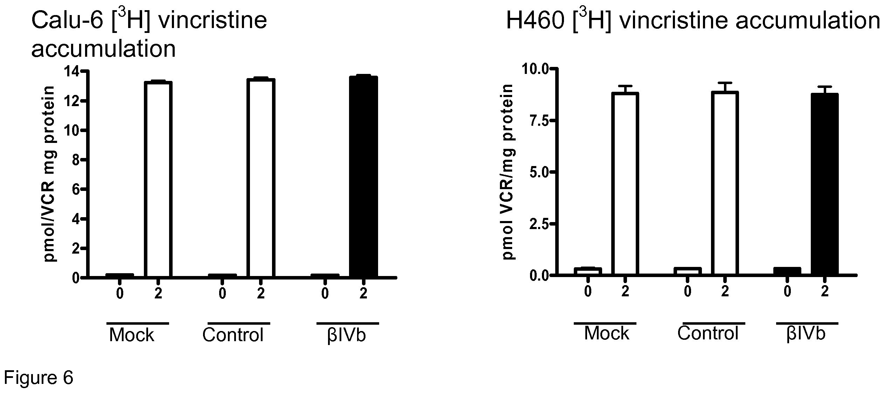

Figure 6 provides graphs which illustrate the cellular uptake and retention of [³H]-vincristine in mock-transfected, control siRNA-transfected and βIVb siRNA-transfected Calu-6 and H460 NSCLC cells. The retention of [³H]-vincristine was determined at the time of addition (zero time) and after 2 h. There was no significant difference in the intracellular [³H]-vincristine content between the βIVb knockdown and control cells. Open bars: mock and control siRNA-transfected cells; solid bars: βIVb siRNA-transfected cells. Values shown are the mean ± SEM of at least four separate experiments.

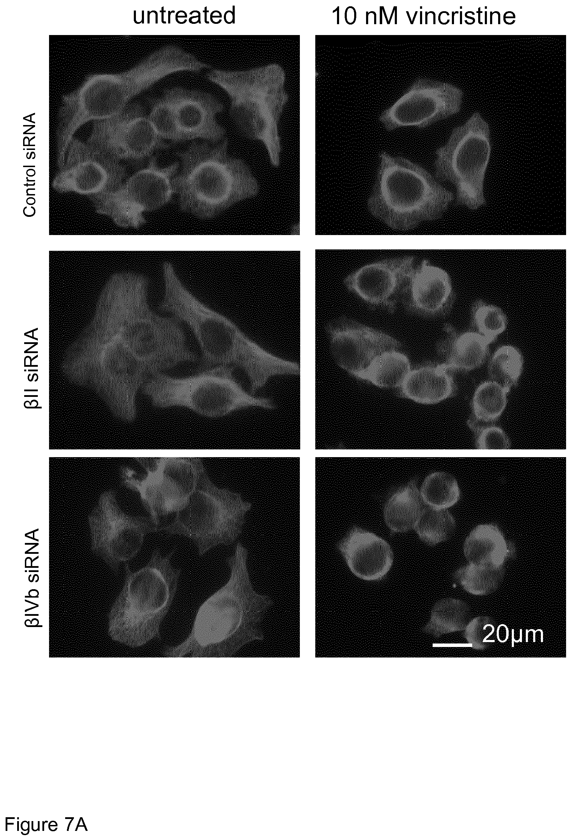

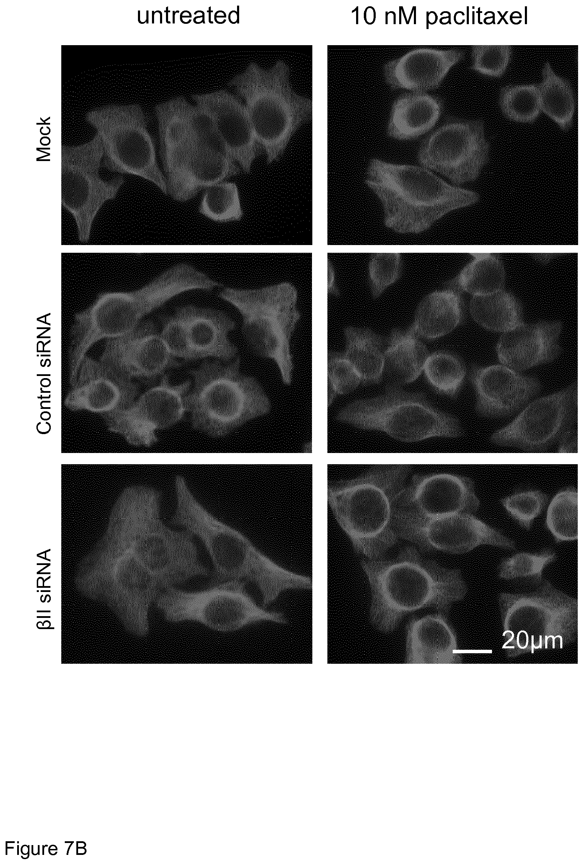

Figure 7 provides photomicrographs which illustrate the effect of βII or βIVb knockdown on the microtubule network upon treatment with vincristine or paclitaxel. Calu-6 cells were fixed and stained with α-tubulin 72 hours after siRNA transfection. Both βII and βIVb transfectants display a normal microtubule cytoskeleton. Figure 7A illustrates the effect of vincristine treatment on microtubules of βII and βIVb siRNA-transfected cells. When treated with 10 nM of vincristine both βII and βIVb transfectants showed extensive disruption of microtubules compared to the control. Figure 7B illustrates the effect of paclitaxel on the microtubules of βII transfectants. Microtubule networks of βII transfectants were comparable to the controls after incubation with 10 nM paclitaxel.

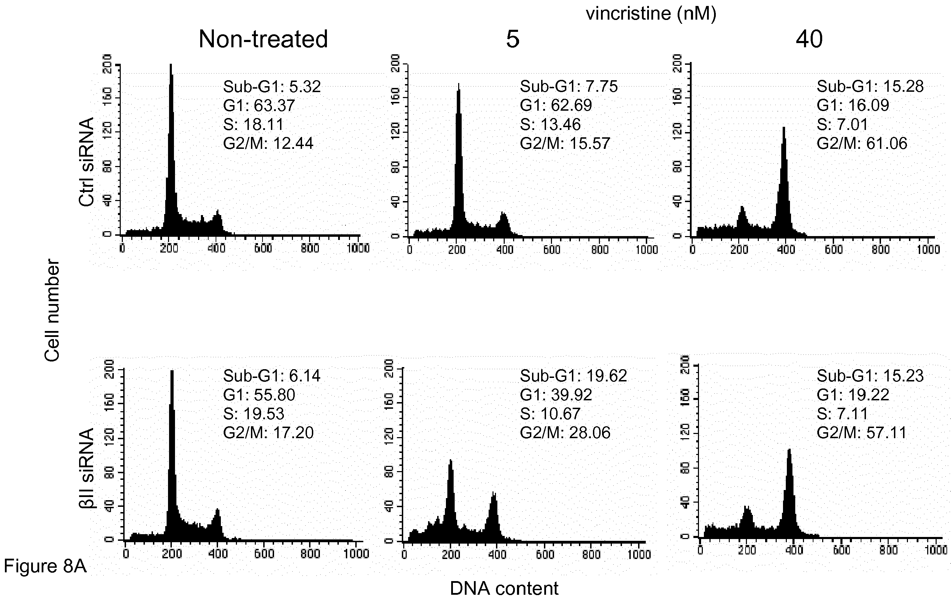

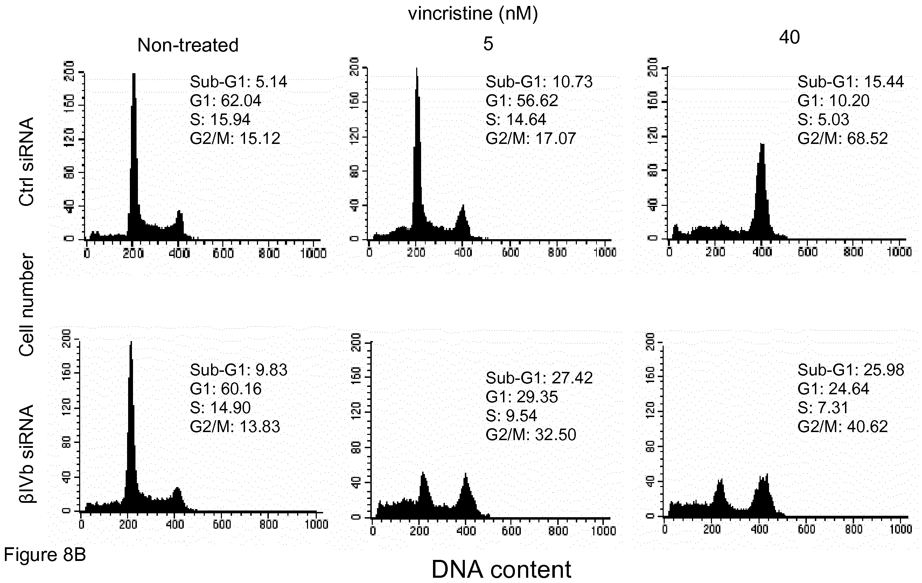

Figure 8 illustrates a cell cycle analysis of βII and βIVb transfected H460 cells after exposure to vincristine. Transfected cells were incubated with vincristine at either 5 or 40nM for 24h; fixed and stained with propidium iodide and analysed by flow cytometry. Figure 8A demonstrates that βII transfectants showed a comparable G₂-M accumulation to the controls. Figure 8B demonstrates that βIVb depletion abolished the accumulation of cells in G₂-M following vincristine treatment at higher concentration, and instead promoted an increase in the sub-G₁ fraction of cells. The histograms which are shown are representative of three experiments performed.

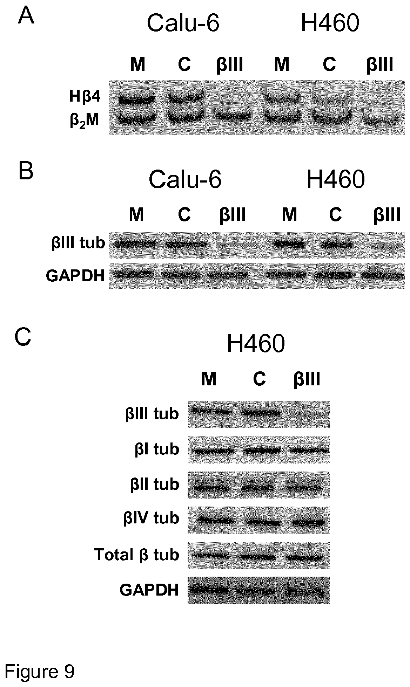

Figure 9A shows analysis of βIII-tubulin gene expression by RT-PCR following 48 h of βIII-tubulin siRNA transfection at 25 nmol/L (Calu-6) or 100 nmol/L (H460). The expression of the β2-Microglobulin β₂M) "housekeeping" gene served as an internal control. Figure 9B shows protein expression of βIII-tubulin 72 h after βIII-tubulin transfection. The expression of the "housekeeping" GAPDH polypeptide was used as a loading control. Figure 9C illustrates specificity of βIII-tubulin siRNA. No significant changes were observed in the expression of class I, II, and IV β-tubulin isotypes and total β-tubulin at the protein level. *M,* mock; C, control-siRNA; *βIII*, βIII-tubulin siRNA.

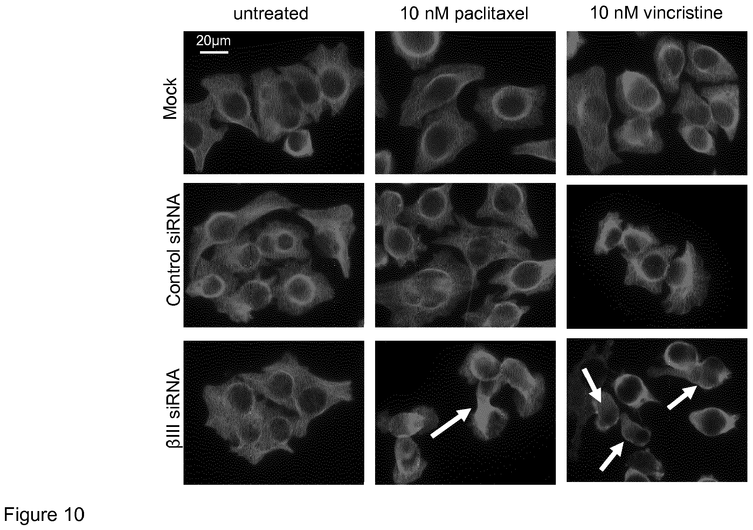

Figure 10 illustrates microtubule morphology in siRNA-transfected Calu-6 cells incubated with 10 nmol/L of paclitaxel (*middle*) and 10 nmol/L of vincristine (*right*) for 1 h. Microtubules are shown by α-tubulin inmmunofluorescent staining. Extensive microtubule disruption occurred when βIII transfectants were treated with either paclitaxel or vincristine. Cells with abnormal morphology are marked with arrows.

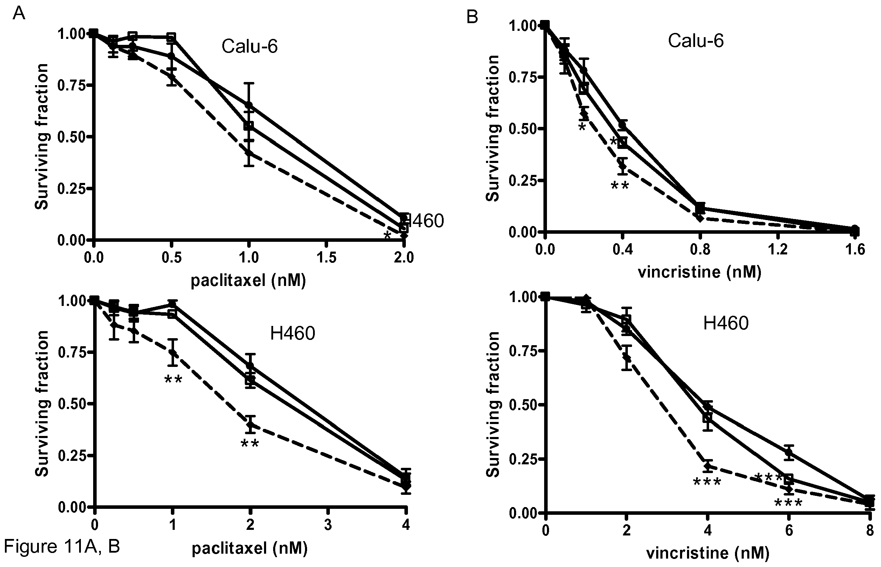

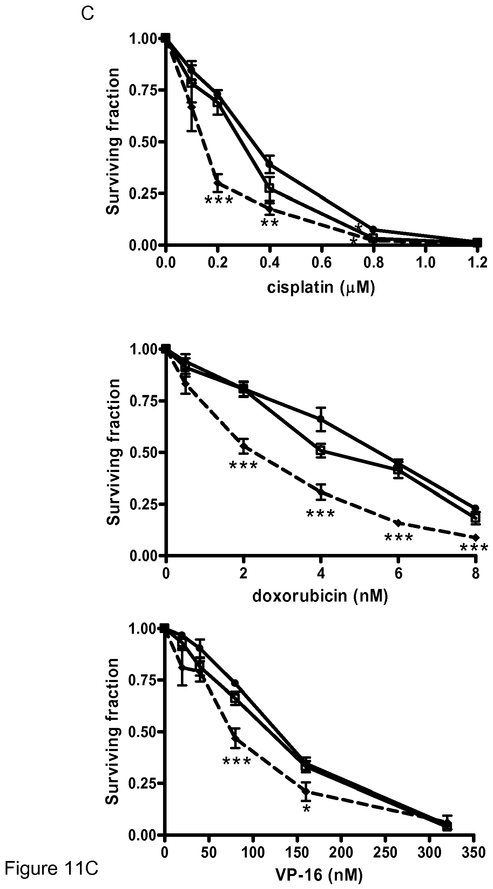

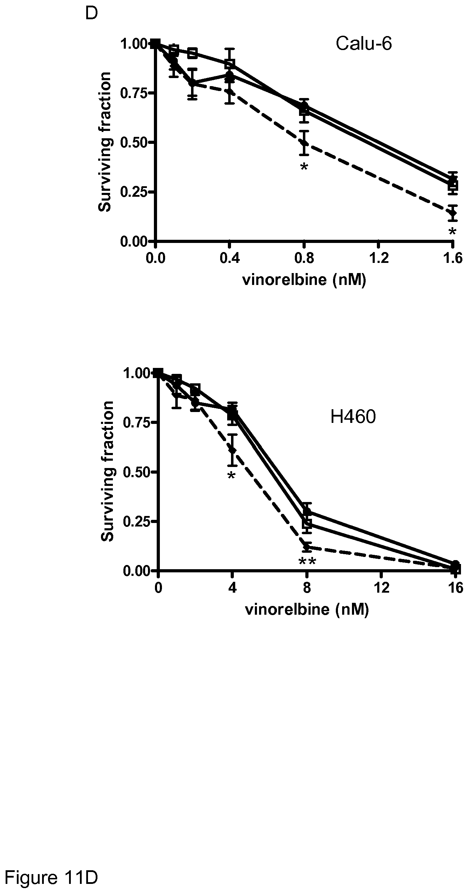

Figure 11 shows results of clonogenic assays in the presence of tubulin-binding agents or DNA-damaging agents. Clonogenic assays were done on mock (closed circles, solid lines), control siRNA (open squares, solid lines), and βIII-transfected cells (closed diamond, dashed lines). Figure 11A illustrates clonogenic survival, expressed as surviving fractions, for treatment with paclitaxel. Figure 11B illustrates clonogenic survival, expressed as surviving fractions, for treatment with vincristine. Figure 11C illustrates clonogenic survival, expressed as surviving fractions, for treatment with DNA-damaging agents such as cisplatin (top), doxorubicin (middle), and etoposide (VP-16; bottom). Figure 11D illustrates clonogenic survival, expressed as surviving fractions, for treatment of Calu-6 (top) and H460 cells (bottom) with vinorelbine. Each of the data points represents the mean of at least four individual assays. The bars represent the standard error of the mean. Statistics were calculated by comparing the surviving fraction of the siRNA-treated cells with the mock-transfected cells at each drug concentration. *, P < 0.05; **, P < 0.005; ***, P < 0.005.

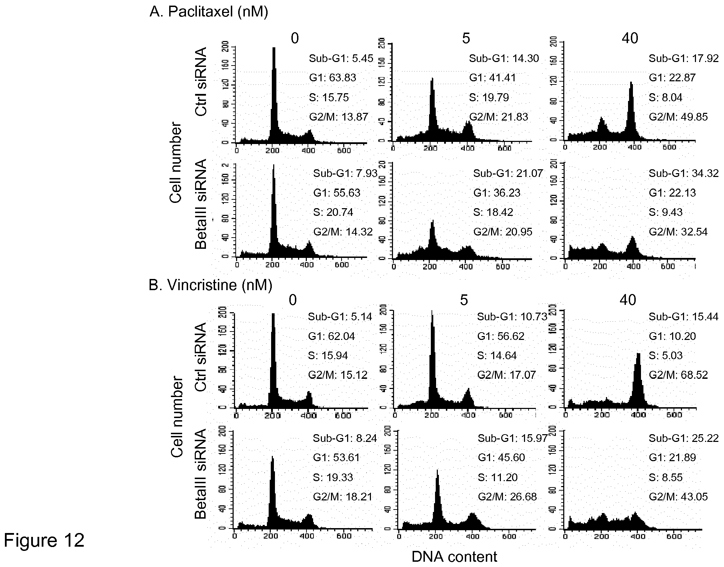

Figure 12 illustrates cell cycle analysis of βIII-tubulin-depleted H460 cells treated with paclitaxel (A) or vincristine (B). Cells were harvested after 24 h of drug treatment and subsequently assayed for their DNA content by flow cytometry. Representative figures of multiple experiments are shown.

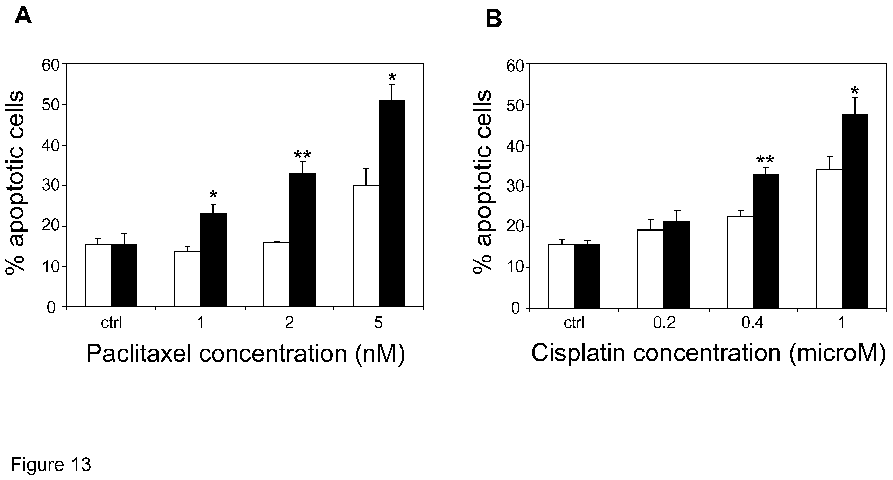

Figures 13A and 13B are graphs showing induction of apoptosis in control siRNA-transfected (white columns) and βIII-tubulin siRNA-transfected (black columns) H460 cells after treatment with paclitaxel (Figure 13A) and after treatment with cisplatin (Figure 13B). Cells were harvested after 48 h of incubation with drug and subsequently assayed for apoptosis induction by flow cytometry using Annexin V-FITC staining. Each of the columns represents mean value of at least three independent experiments, whilst the bars represents the standard error of the mean. *, P < 0.05; **, P < 0.01.

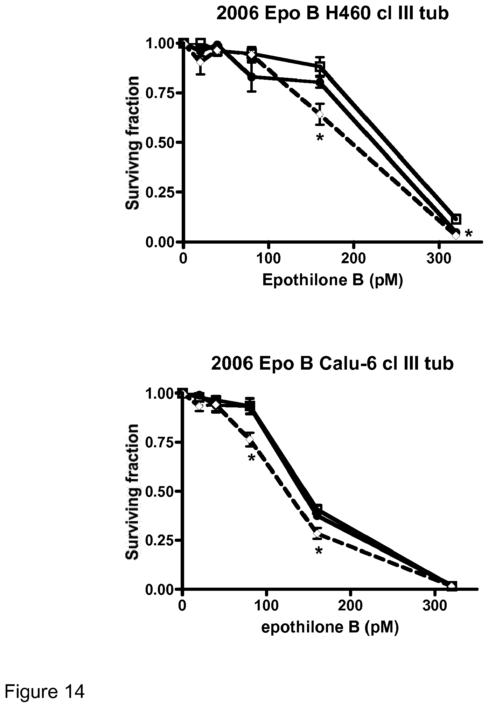

Figure 14 shows results of clonogenic assays in the presence of the tubulin-binding agent epothilone. Clonogenic assays were done on mock (closed circles, solid lines), control siRNA (open squares, solid lines), and βIII-transfected cells (open diamond, dashed lines). Figure 11 illustrates clonogenic survival, expressed as surviving fractions, for treatment of H460 (top) or Calu-6 (bottom) cells with epothilone.

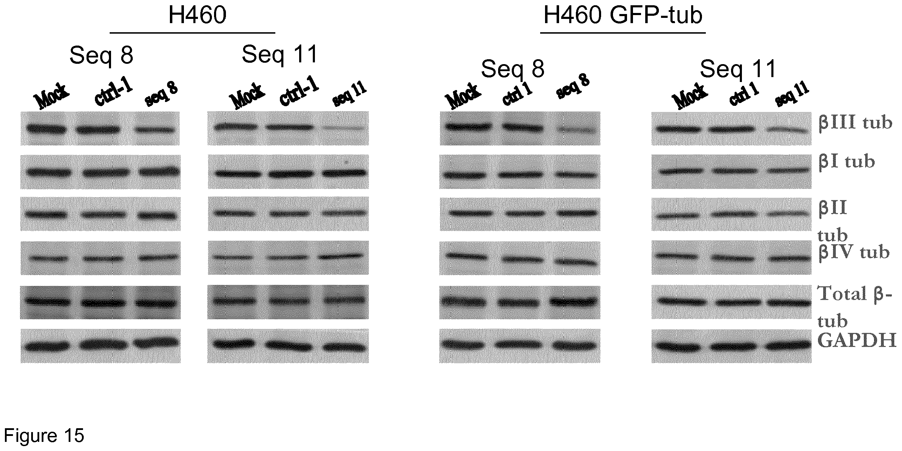

Figure 15 illustrates the specificity of two different 27-mer siRNAs (designated seq 8 and seq 11 in this figure) which were used to knockdown βIII tubulin expression, as demonstrated in western blots. No significant changes in the level of protein expression were observed for other β-tubulin isotypes in βIII H460 knockdown cells.

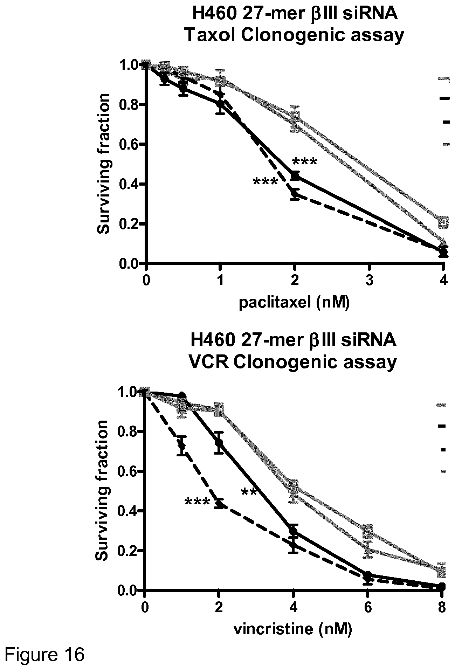

Figure 16 shows the results of clonogenic assays of H460 cells in the presence of the tubulin-binding agents paclitaxel (top) or vincristine (bottom). Clonogenic assays were done on mock (closed triangles, solid lines), control siRNA (open squares, solid lines), and βIII-transfected cells using different 27-mer siRNAs (seq 8 -closed diamond, dashed lines; seq 11 closed squares, solid line). Figure 16 illustrates clonogenic survival, expressed as surviving fractions. An increase in sensitivity to both tubulin-binding agents was demonstrated with each of the different 27-mer siRNAs directed to class III β-tubulin comparing the ID50 values of each of the βIII 27-mer siRNAs to the mock-transfected cells. **, P<0.005, ***, P<0.0005.

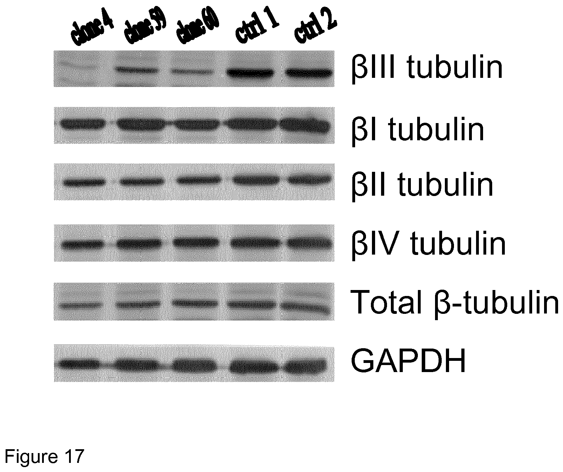

Figure 17 illustrates the specificity of a short hairpin RNA which was introduced into H460 cells to produce three clones in which βIII tubulin expression was stably knocked down (clones 4, 59 and 60). As demonstrated in western blots, no significant changes in the level of protein expression were observed for other β-tubulin isotypes in the βIII H460 knockdown clones. Some variation in the amount of βIII-tubulin protein expression between these stably knocked down clones was observed.

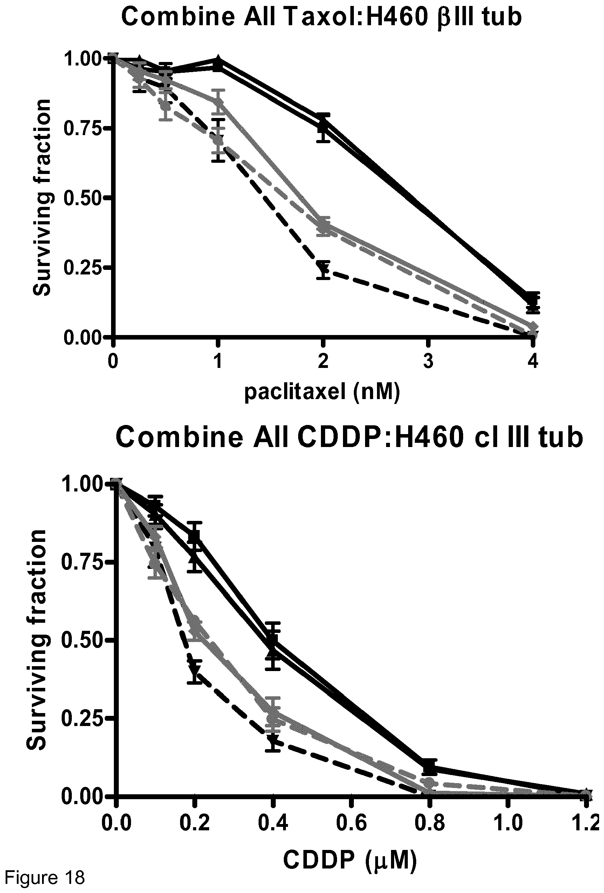

Figure 18 shows the results of clonogenic assays of the clones of H460 cells in which βIII tubulin expression was stably knocked down, in the presence of the tubulin-binding agent paclitaxel (top) or the DNA damaging agent cisplatin (bottom). Clonogenic assays were done on control siRNA (solid squares or solid triangles, solid lines), and three different βIII-stably transfected clones (clone 4 closed down-pointing triangles, dashed lines; clone 59 closed diamonds, solid line; clone 60 closed squares, dashed lines). Figure 18 illustrates clonogenic survival, expressed as surviving fractions. An increase in sensitivity to both paclitaxel and the DNA damaging agent cisplatin was observed in each of the different clones with stable knockdown of βIII-tubulin. The clones with the greatest amount of β111-tubulin knockdown (as demonstrated in Figure 17) appeared to demonstrate the greatest increase in sensitivity to each of the agents.

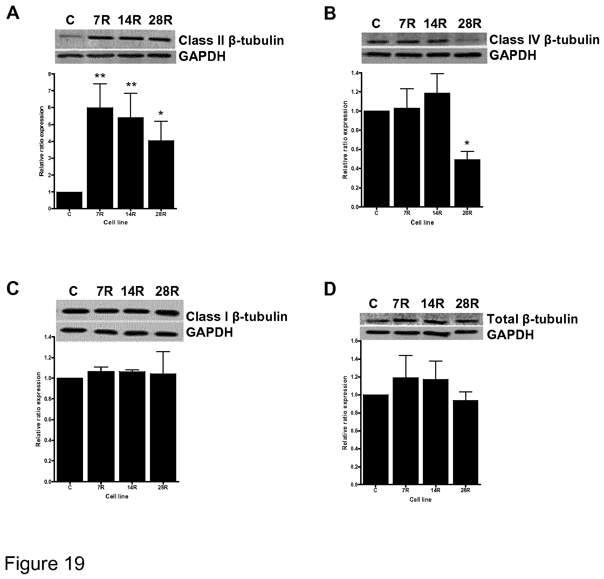

Figure 19 shows the relative tubulin isotype expression of three different leukaemia cell sub-lines (7R, 14R, 28R) which had been selected for resistance to 2-methoxy estradiol. The protein expression demonstrated in the Western blots was plotted as normalized to a control level of expression of the parent cell line. All of the resistant cells exhibited an increase in the expression of class II β-tubulin.

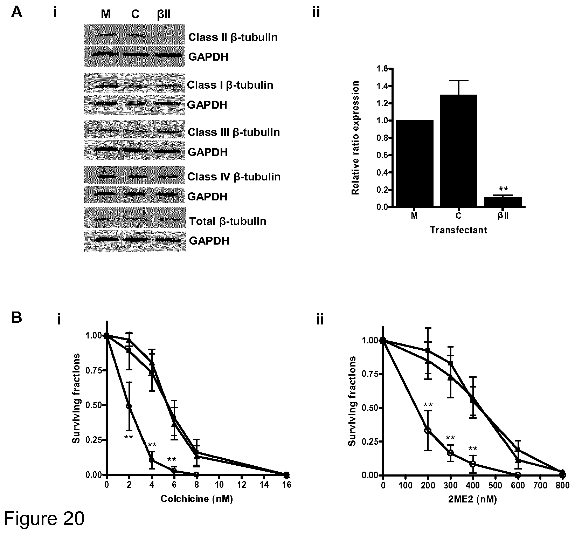

Figure 20 shows the change in the level of class II β-tubulin expression by H460 cells following class II β-tubulin knockdown (A), and the results of clonogenic assays of H460 cells in which class II β-tubulin was knocked down, in the presence of 2-methoxy estradiol or cholchicine (B). Classs II β-tubulin knockdown greatly increased the sensitivity of H460 cells to both 2-methoxy estradiol and cholchicine.

### Detailed Description of the Preferred Embodiments

The invention provides nucleic acid constructs, siRNA molecules and pharmaceutical compositions for use, as defined in the claims.

Methods of screening for the resistance of a tumour cell to a tubulin-binding agent. Such methods comprise detecting the expression of any one or more of class II, class III and class IVb β-tubulin by the tumour cell,
wherein expression of any one or more of class II, class III and class IVb β-tubulin by the tumour cell is predictive of the resistance to or potential resistance to the tubulin-binding agent.

A method of assessing the resistance of a tumour cell to a tubulin-binding agent is disclosed. The method includes detecting an amount of a class II, class III and class IVb β-tubulin protein or a class II, class III and class IVb β-tubulin mRNA by the tumour cell. A subject tumour cell tubulin amount is thereby obtained. The subject tumour cell tubulin amount is compared to a control cell tubulin amount thereby determining a tubulin amount difference. The control cell tubulin amount is a respective amount of a class II, class III or class IVb β-tubulin protein or any one or more of class II, class III and class IVb β-tubulin mRNA in a control cell. The resistance of the tumour cell to the tubulin-binding agent is assessed based on the tubulin amount difference.

A "subject tumour cell" is simply a tumour cell derived from a sample tumour that is the subject of the resistance assessment. And as indicated above, a subject tumour cell tubulin amount is simply the amount of any one or more of a class II, class III and class IVb β-tubulin protein or a class II, class III and class IVb β-tubulin mRNA detected in the method.

A "control cell" is a cell with a known amount of a class II, class III and/or class IVb β-tubulin protein or a class II, class III and/or class IVb β-tubulin mRNA and a known level of resistance to the tubulin binding agent. In some embodiments, the control cell is a control tumour cell, discussed below. Control cells may be obtained from organisms (or organs) or derived using methods of the present invention. The control cell may be part of a dose response curve generated from exposing cells to a plurality of difference dosages of the tubulin binding agent. Moreover, the method of assessing the resistance of a tumour cell may itself be used to generate such a dose response curve. In some embodiments, the control cell is a mammalian control cell such as a human control cell.

The control cell tubulin amount is the amount of a class II, class III and/or class IVb β-tubulin protein or a class II, class III and/or class IVb β-tubulin mRNA respective to the subject tumour cell tubulin amount. For example, where the subject tumour cell tubulin amount is an amount of class II and class III β-tubulin protein detected from the subject tumour cell, the control cell tubulin amount is the amount of class II and class III β-tubulin within the control cell.

As is apparent from the description of the method of assessing the resistance of a tumour cell above, a "tubulin amount difference" is simply the difference between the subject tumour cell tubulin amount and the control cell tubulin amount.

As is apparent from the description of the methods herein, where the subject tumour cell tubulin amount is higher than the control cell tubulin amount, in some embodiments the subject tumour cell would be assessed as having greater resistance to the tubulin-binding agent than the control cell. For example, where the subject tumour cell class III tubulin amount is higher than the control cell tubulin amount, the subject cell would be assessed as having greater resistance (and less sensitivity) to a vinca alkaloid, a taxane, an epothilone or a DNA damaging agent. For example, where the subject tumour cell class II tubulin amount is higher than the control cell tubulin amount, the subject cell would be assessed as having greater resistance (and less sensitivity) to a vinca alkaloid or 2-methoxy estradiol. For example, where the subject tumour cell class IVb tubulin amount is higher than the control cell tubulin amount, the subject cell would be assessed as having greater resistance (and less sensitivity) to a vinca alkaloid, and to have less resistance (and greater sensitivity) to an epothilone.

Conversely, where the subject tumour cell tubulin amount is lower than the control cell tubulin amount, in some embodiments the subject tumour cell may be assessed as having less resistance (and greater sensitivity) to the tubulin-binding agent than the control cell. For example, where the subject tumour cell class III tubulin amount is lower than the control cell tubulin amount, the subject cell would be assessed as having less resistance (and greater sensitivity) to a vinca alkaloid, a taxane, an epothilone or a DNA damaging agent. For example, where the subject tumour cell class II tubulin amount is lower than the control cell tubulin amount, the subject cell would be assessed as having lower resistance (and greater sensitivity) to a vinca alkaloid or 2-methoxy estradiol. For example, where the subject tumour cell class IVb tubulin amount is lower than the control cell tubulin amount, the subject cell would be assessed as having lower resistance (and greater sensitivity) to a vinca alkaloid, and to have greater resistance (and lower sensitivity) to an epothilone.

Control tumour cells of known resistance to the agent may be, for example, tumour cells which are isolated from an individual prior to successful treatment with the agent. A series of control tumour cells of a range of known resistances to the agent may be assembled for example from tumour cells which are isolated from a group of individuals prior to successful and unsuccessful treatments with the agent.

As demonstrated herein, the presence of certain classes of tubulin in a tumour cell contributes to the responsiveness of the tumour cell to the administration of anti-mitotic agents, such as tubulin-binding agents, or DNA-damaging agents. Accordingly, the assessment of expression of any one or more of class II, class III and class IVb β-tubulin by the tumour cell provides guidance as to the tubulin-dependent responsiveness of the tumour cell to different tubulin-binding agents or DNA-damaging agents, and provides a method by which agents to treat the tumour may be selected.

The resistance of a tumour cell to a tubulin-binding agent may be a complete resistance, or it may be a partial resistance. A tumour cell may be considered "resistant" to a tubulin-binding agent if it is unresponsive to the administration of a tubulin binding agent. An unresponsive tumour cell may demonstrate, for example, an unchanged rate of cell division, an unchanged rate of apoptosis or unchanged cell morphology on exposure to the tubulin binding agent at concentrations which are cytotoxic to non-tumour cells or tumour cells which are sensitive to the agent.

The tumour cell may be resistant to the administration of the tubulin-binding agent *in vitro.* The tumour cell may be resistant to the administration of the tubulin-binding agent *in situ.*

In some embodiments a tumour cell may be considered "resistant" to a tubulin-binding agent if it demonstrates a reduced response to the administration of a tubulin-binding agent when compared to control tumour cells of known resistance to the agent.

Control tumour cells from a single tumour with a range of resistances to a tubulin-binding agent and a range of tubulin expression may be generated for the purpose of providing control cells for comparison purposes. Cells with a range of sensitivities may be generated for example by taking control tumour cells which are known to be resistant to the agent, dividing the cells into groups, and stably modulating the expression of at least one of class II, class III and class IVb β-tubulin in each of the groups of cells, using for example the siRNA or shRNA methods described herein. By generating different groups of cells in which the expression of at least one of class II, class III and class IVb is stably reduced in a controlled fashion, as demonstrated herein it is possible to generate cells from a single tumour with a range of sensitivities to a tubulin-binding agent.

The resistance of a tumour may be assessed with reference to concentration of a tubulin-binding agent which is used therapeutically in human subjects.

In some embodiments a tumour cell may be considered "resistant" to a tubulin-binding agent if it demonstrates a response to a tubulin-binding agent which is similar to the response of cells of a control tumour of known resistance to the tubulin-binding agent. A control tumour of known resistance to a tubulin-binding agent may be isolated, for example, from a subject prior to the unsuccessful treatment with the tubulin-binding agent. Alternatively, a resistant control tumour cell may be generated from a primary tumour cell or a tumour cell line *in vitro* by treating a tumour primary tumour cell or tumour cell line at least once with the tubulin-binding agent at a concentration which results in greater than 90% cell death, and selecting for cells which survive the treatment.

A tumour cell may be "potentially resistant" if it is suspected of resistance to or of becoming resistant to a microtubule-binding agent.

The method comprises "detecting" the expression of any one or more of class II, class III and class IVb β-tubulin by the tumour cell. In certain embodiments the identification of the presence of a particular tubulin in tumour cells as detected by a particular technique may be sufficient to assess the tumour as being resistant to a tubulin-binding agent, and conversely the identification of the absence of a particular tubulin in tumour cells by the technique may be sufficient to assess the tumour as being sensitive to the agent. In such embodiments it may not be necessary to make a determination of the amount of tubulin expressed by the tumour cells in order to assess the resistance of the tumour cell.

In other embodiments, the detecting of the expression of any one or more of class II, class III and class IVb β-tubulin by the tumour cell will involve a comparison of the amounts of tubulin of any one or more of these classes expressed by the tumour cell with the amount of tubulin of any one or more of these classes expressed by at least one cell of known resistance.

In particular embodiments the comparison will be with the amount of tubulin expressed by a plurality of control cells of different known resistances. As demonstrated in the examples provided herein, in at least certain embodiments the degree or resistance of a tumour cell may be proportional (for example in the case of class III β-tubulin and vinca alkaloids) or inversely proportional (for example in the case of class IVb β-tubulin and epothilone) to the amount of tubulin which is expressed by the tumour cell. Accordingly, a comparison may be made against a dose-response curve generated for example from multiple control tumour cells.

Thus, the detecting of expression of any one or more of class II, class III and class IVb β-tubulin by the tumour cell may comprise detecting the presence or absence of tubulin mRNA or the presence or absence of tubulin polypeptide in the tumour cell.

Alternatively, the detecting of expression of any one or more of class II, class III and class IVb β-tubulin by the tumour cell may comprise determining the relative amount of tubulin mRNA or the relative amount of tubulin polypeptide expressed by the tumour cell.

The relative amount may be in comparison with the abundance of a tubulin mRNA or the amount of a tubulin polypeptide in cells of a known control sensitive tumour. The relative amount may be in comparison with the amount of a tubulin mRNA or the amount of a tubulin polypeptide in cells of a known resistant control tumour. The relative amount may be in comparison with the amount of a tubulin mRNA or the amount of a tubulin polypeptide in control tumour cells of a range of sensitivities. The relative amount may be in comparison with the amount of tubulin mRNA or the amount of tubulin polypeptide in non-tumour cells taken from the tissue surrounding the tumour. The relative amount may be a normalised amount as determined by

The presence or absence or relative amount of tubulin mRNA may be detected by any one or more of RT-PCR, quantitative PCR, semi-quantitative PCR, or *in-situ* hybridization under stringent conditions, using one or more probes or primers which are specific for any one of class II, class III and class IVb β-tubulins. Examples of polynucleotides which may be used for RT-PCR or semi-quantitative PCR techniques are described in Kavallaris et al. (1997) J Clin Invest 100, 1282-1293.. In a particular embodiment, the presence or absence of mRNA is detected using RT-PCR, for example using methodology and specific primers as described in Kavallaris et al. (1997) J Clin Invest 100, 1282-1293.

Where a measurement of the relative amount of a tubulin mRNA is required, known techniques such as real-time reverse transcriptase polymerase chain reaction may be employed to reverse transcribe RNA and then quantitate the resulting cDNA using real-time PCR. Quantitative techniques for assessing the expression of RNA are reviewed in Ding and Cantor (2004) JBiochem Mol Biol 37(1):1-10, and in Bustin et al., (2005) Journal of Molecular Endocrinology 34: 579-601.

The presence or absence or relative amount of tubulin polypeptide may be detected using any one or more of Western blotting, ELISA, or other standard quantitative or semi-quantitative techniques available in the art, or a combination of such techniques for identifying the presence of specific polypeptides. A range of quantitative and semi-quantitative proteomic techniques are reviewed, for example in Hirsch et al., (2004) Am J Physiol Lung Cell Mol Physiol 278: L1-L23.. Techniques relying on antibody recognition of one or more specific tubulin isotype polypeptides are contemplated in particular. In a particular embodiment, the presence or absence or relative abundance of tubulin polypeptide may be detected with techniques which comprise semi-quantitative Western blotting, for example using the Western blotting technique described in Example 2 described herein. In other particular embodiments, the presence or absence or relative abundance of tubulin polypeptide may be detected with techniques which comprise antibody capture of tubulin polypeptides in combination with electrophoretic resolution of captured tubulin polypeptides, for example using the Isonostic ™ Assay (Target Discovery, Inc.).

In particular embodiments, tumour cells which are detected as expressing β-III tubulin are predicted to be resistant to a vinca alkaloid, a taxane, an epothilone or a DNA damaging agent.

In particular embodiments, tumour cells which are detected as expressing β-II tubulin are predicted to be resistant to a vinca alkaloid or 2-methoxy estradiol.

In particular embodiments, tumour cells which are detected as expressing β-IVb tubulin are predicted to be resistant to a vinca alkaloid, and to be sensitive to an epothilone.

In certain embodiments, the tubulin-binding agent is a microtubule stabilizing agent, such as a taxane or an epothilone.

In certain embodiments, the tubulin-binding agent is a microtubule destabilizing agent, such as a vinca alkaloid or 2-methoxy estradiol.

Methods described herein of screening for or assessing the resistance of a tumour cell to a tubulin-binding agent may be practiced prior to the administration of an anti cancer agent, in order to determine whether the cells of the tumour will respond to one or more tubulin-binding agents, or may be practiced during a course of treatment, to determine whether the cells of the tumour are developing resistance to the tubulin-binding agent.

The introduction of a nucleic acid construct comprising a nucleic acid sequence specific for at least a portion of the class II, class III or IVb β-tubulin gene to a tumour, wherein the nucleic acid construct decreases the expression of class II, class III or IVb β-tubulin is also disclosed. As noted above the invention provides nucleic acid constructs, siRNA molecules and pharmaceutical compositions, for use as defined in the claims.

Although the exemplified nucleic acid constructs comprising a nucleotide sequence described herein are siRNA or shRNA sequences, it will be clearly understood that the targeted disruption of the expression of class II, class III and/or IVb β-tubulin genes may be achieved using any molecules which selectively target and inhibit the expression of class II, class III and/or IVb β-tubulin genes, such as antisense sequences, siRNA sequences, shRNA sequences, ribozyme sequences and the like. The "expression" of class II, class III and/or IVb β-tubulin genes is intended to encompass the transcription and/or translation of class II, class III and/or IVb β-tubulin sequences. Accordingly, the nucleic acid sequence which is specific for at least a portion of the class II, class III or IVb β-tubulin gene is also intended to encompass a nucleic acid sequence which is specific for at least a portion of the class II, class III or IVb β-tubulin mRNA.

"Detecting the expression" is intended to encompass not only the detection of the presence of class II, class III or IVb β-tubulin mRNA or protein but also in certain embodiments the amount of the class II, class III or IVb β-tubulin mRNA or protein.

"Detecting the amount" of a class II, class III or IVb β-tubulin mRNA or protein is intended to include detecting the absolute levels of the mRNA or protein, or in certain embodiments the relative amounts of the mRNA or protein. The relative amounts are relative to one or more other cellular proteins or mRNAs, such as other tubulin proteins or mRNAs or a housekeeping gene protein or mRNA in the cell. Thus in some embodiments the amounts are normalised relative to other cellular proteins or mRNAs.

Methods for the design, synthesis, and delivery of antisense nucleic acids are well known in the art. The antisense molecules may be DNA or RNA, or partial or complete synthetic analogues thereof. Antisense constructs may be generated which are at least substantially complementary along their length to the region of the gene in question. Binding of an antisense construct to its complementary cellular sequence may interfere with transcription, RNA processing, transport, translation and/or mRNA stability.

Suitable antisense oligonucleotides may be prepared by methods well known to those of skill in the art. Typically antisense oligonucleotides will be synthesized on automated synthesizers. Suitable antisense oligonucleotides may include modifications designed to improve their delivery into cells, their stability once inside a cell, and/or their binding to the appropriate target. For example, the antisense oligonucleotide may be modified by the addition of one or more phosphorothioate linkages, or the inclusion of one or more morpholine rings into the backbone. The antisense oligonucleotide may be 10-30 base pairs in length and will be target regions of the class II, class III and/or IVb β-tubulin gene. In one embodiment, the antisense oligonucleotide sequences will have no more than 90% sequence identity with other known tubulin isotypes.

As a practical matter, whether any particular nucleic acid molecule is no more than 90% identical to, for instance, the nucleotide sequence of other known tubulin isotypes can be determined conventionally using known computer programs such as the Bestfit program (Wisconsin Sequence Analysis Package, Version 8 for Unix, Genetics Computer Group, University Research Park, 575 Science Drive, Madison, Wis. 53711). Bestfit uses the local homology algorithm of Smith and Waterman to find the best segment of homology between two sequences (Advances in Applied Mathematics 2:482-489 (1981)). When using Bestfit or any other sequence alignment program to determine whether a particular sequence is, for instance, 90% identical to a reference sequence, the parameters are set such that the percentage of identity is calculated over the full length of the reference nucleotide sequence and that gaps in homology of up to 5% of the total number of nucleotides in the reference sequence are allowed. A preferred method for determining the best overall match between a query sequence and a subject sequence, also referred to as a global sequence alignment, can be determined using the FASTDB computer program based on the algorithm of Brutlag and colleagues (Comp. App. Biosci. 6:237-245 (1990)). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identity are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the length of the subject nucleotide sequence, whichever is shorter.

If the subject sequence is shorter than the query sequence because of 5' or 3' deletions, not because of internal deletions, a manual correction must be made to the results. This is because the FASTDB program does not account for 5' and 3' truncations of the subject sequence when calculating percent identity. For subject sequences truncated at the 5' or 3' ends, relative to the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. Only bases outside the 5' and 3' bases of the subject sequence, as displayed by the FASTDB alignment, which are not matched/aligned with the query sequence, are calculated for the purposes of manually adjusting the percent identity score.

It may be advantageous if the specific sequences to be targeted are unique to either class II, class III and/or IVb β-tubulin gene sequences. These amino acid sequences of human β-tubulin isotypes are described in Verdier-Pinard et al., (2005) Biochemistry 44:15858-15870.Corresponding cDNA and/or genomic DNA sequences are also known. The skilled addressee will be able to readily determine whether any given sequence is specific to either class II, III and/or IVb β-tubulin gene sequences.

Small interfering RNA (siRNA) sequences are small, usually double-stranded RNA oligonucleotides, for example at 21, 27 or 29 bases in length with or without overhangs, which specifically hybridise with RNA sequences of interest and which serve as substrates for the RNA-induced silencing complex. Double-stranded RNA molecules may be synthesised in which one strand is identical to a specific region of the mRNA transcript to be silenced, and this double stranded RNA may be introduced directly. Alternatively, corresponding dsDNA can be employed, which, once presented intracellularly is converted into dsRNA. Methods for the synthesis of suitable siRNA molecules for use in RNA interference (RNAi) and for achieving post-transcriptional gene silencing are known to those of skill in the art, and there are now commercial services for designing and producing siRNAs.

The skilled addressee will appreciate that a range of suitable siRNA constructs capable of inhibiting the expression of the class II, class III and/or class IVb β-tubulin can be identified and generated based on knowledge of the sequence of the genes in question using routine procedures known to those skilled in the art without undue experimentation. As demonstrated herein, a variety of siRNA or shRNA sequences directed to different nonoverlapping regions of a specific β-tubulin are able to influence the expression of that tubulin in a cell. Those skilled in the art will appreciate that there need not necessarily be 100% nucleotide sequence match between the target sequence and the siRNA sequence. The capacity for mismatch is dependent largely on the location of the mismatch within the sequences. In some instances, mismatches of 2 or 3 nucleotides may be acceptable but in other instances a single nucleotide mismatch is enough to negate the effectiveness of the siRNA. The suitability of a particular siRNA molecule may be determined using routine procedures known to those skilled in the art without undue experimentation.

Although the maximal effects of antisense nucleic acids and siRNA on the specific inhibition of RNA or protein expression are comparable, siRNA generally produces a longer-lasting effect. siRNAs may be introduced into a cell by way of a vector, for example via a viral-mediated delivery mechanism such as an adeno-associated virus vector, or delivered exogenously, for example when delivered as part of a liposome complex. Techniques for the non-viral delivery of synthetic *siRNAs in vivo* are reviewed in Akhtar and Benter (2007) J. Clin. Invest 117:3623-3632. Techniques for the administration of siRNA sequences locally or systemically to mice (Yano et al, (2004) Clinical Cancer Research 10: 7721-7726), to primates (Zimmermann et al, (2006) Nature 441(7089): 111-114) and to humans (Nogawa et al. (2006) J Clin Invest 115:978-985) have been described (see for example the review of Akhtar and Benter (2007) J Clin Invest 117: 3623-3632), and these have demonstrated that this class of molecules can be used to reduce the expression of target *genes in vivo.* The use of siRNA delivery systems such as cholesterol-siRNA conjugates, cationic delivery systems, including cationic nanoparticles or cationic liposomes or cationic polymer or peptide delivery systems, or chitosan-siRNA conjugates is contemplated.

The systemic administration of siRNA has been demonstrated to result in an accumulation of the siRNA in the reticuloendothelial system, including the liver and lungs, and accordingly this may be advantageous when administering an siRNA for the treatment of lung tumours.

Ribozymes, such as hammerhead or hairpin ribozymes, are capable of the targeted catalytic cleavage and splicing of specific RNA sequences, including mRNA and genomic RNA sequences. The design and methods for the delivery of ribozymes are reviewed, for example, in Vaish, Kore and Eckstein (1998) Nucleic Acids Research 26:5237-5242; in Lieber and Strauss (1995) Mol. Cell. Biol. 15:540-551; and in Usman and Blatt (2000) J Clin Invest 106:1197-1202. The use of ribozymes to target and regress specific tumours is described in the art, for example Zhang et al. (2000) Gene Their 7:2041-2050.

In general, each of these classes of molecules appears to be well tolerated, either when administered to human or animal subjects systemically, such as via intravenous routes, as a bolus or by sustained release techniques subcutaneously, or when directly applied to tumour cells or injected into or in the vicinity of tumours.

Delivery to the lungs in a formulation which comprises an inhalable polynucleotide construct is also contemplated, in particular for tumours of the lung. The administration of siRNA as an aerosol directly to the lungs by nebulizer for the treatment of respiratory syncytial virus infection is currently in clinical trials (Alnylam ALN-RSVOl, Bitco et al, (2005) Nat Med 11:50-55), and has demonstrated safety, tolerance and efficacy in antiviral activity. Accordingly, the delivery of an siRNA to the lungs to modulate specific tubulin expression in tumours of the lungs is contemplated.

Suitable polynucleotide sequences may be administered in isolation or in a nucleic acid construct. The construct may be a plasmid vector, a viral vector, or any other suitable vehicle adapted for the insertion of foreign sequences and introduction into eukaryotic cells. The vector may be an expression vector capable of directing the transcription of a DNA sequence into RNA. Viral expression vectors include, for example, epstein-barr virus-, bovine papilloma virus-, adenovirus- and adeno-associated virus-based vectors.

In one embodiment, the vector is episomal. The use of a suitable episomal vector provides a means of maintaining the polynucleotide sequence in target cells in high copy number extra-chromosomally, thereby eliminating potential effects of chromosomal integration.

In the context of the present invention and disclosure, it may be preferable to administer the polynucleotide construct in a form so that it is not permanently incorporated into the genome of the somatic cell to which it is administered, to allow temporal control of the inhibition of expression of class II, class III and/or class IVb β-tubulin.

Throughout the specification, where reference is made to apolynucleotide, such as a polynucleotide construct or a nucleic acid sequence, it will be understood that it is intended to encompass non-naturally occurring polynucleotides and nucleic acids, provided that they retain the ability to interact specifically in the cell while possessing low toxicity. The use of certain non-naturally occurring nucleic acids, for example, provides increased resistance to nuclease digestion, which in turn may the increase half-life of the nucleic acid following administration. Chemical stabilisation to reduce nuclease digestion, for example, has been demonstrated in the ribozyme "ANGIOZYME" which is currently undergoing clinical trials.

Where exogenous polynucleotide constructs are to be delivered to isolated cells or to a subject, they will be formulated in an appropriate diluent, such as saline. In order to promote translocation of the nucleic acid constructs into the cell cytoplasm the constructs may be encapsulated in liposomes, and/or conjugated to ligands or targeting molecules which promote the recognition of the desired target cells and/or the penetration of the plasma membrane. The use of siRNA conjugated with cell-penetrating peptides, for example, has been demonstrated to increase cellular uptake of siRNA (Veldhoen et al. (2006) Nucleic Acids Res 34: 6561-6573). Other techniques which are known in the art for increasing the transfection efficiency of an applied nucleic acid include biochemical methods, such as the use of DEAE-dextran, the conjugation of the siRNA to nanoparticles, calcium phosphate transfection methods and/or physical transfection methods such as direct micro-injection, electroporation or biolistic particle delivery.

The introduction of a polynucleotide construct as described herein encompasses the use of polycationic agents, which are compounds that can be used to delivery siRNA to cancer cells. As disclosed herein, the polycationic agent polyethylenimine (PEI), which is a low molecular weight compound, may be used as a delivery vehicle for the delivery of the nucleic acid construct. PEI has the ability to deliver siRNA, for example, to cells by firstly, condensing siRNA into positively charged particles which are capable of interacting with anionic proteoglycans at the cell surface and promoting the entry into cells by endocytosis. Secondly, the noncovalent complexation of siRNA with PEI efficiently stabilises the siRNAs and provides greater efficiency of action for a given concentration. PEI is present as a delivery vehicle according to the invention.

As demonstrated herein, specific β-tubulin isotypes confer altered sensitivity to tubulin-binding agents and DNA-damaging agents. This information may be used to exploit natural differences in isotype composition among various cell types to target specific drugs to specific tumours. The hypersensitivity of a cell may result both from enhanced apoptotic pathways and from defective mitosis due to modifications in microtubules and their associated protein, thus enhancing the sensitivity of these cells to tubulin-binding agents.

Although the use of a nucleic acid construct comprising a polynucleotide sequence which is specific to class II and/or class IVb β-tubulin gene to enhance the sensitivity of certain NSCLC cell lines and leukaemia cell lines to tubulin-binding agents is described herein, due to the specificity of the mode of action of the nucleic acid construct it is anticipated that any other tumour cell types which express the class II, class III and/or class IVb β-tubulin genes will also demonstrate an enhanced sensitivity to at least one microtubule destabilizing agent when treated in a similar fashion. For example, ovarian tumours resistant to paclitaxel may express increased levels of class II and/or class IVb β-tubulin, neuroblastoma cells may express high levels of class II, and leukaemia cells and breast cancer cells may also express class II and/or class IVb β-tubulin. As used herein a tumour cell is a neoplastic cell, and is intended to encompass not only cells found in solid tumours but also isolated neoplastic cells or circulating neoplasms such as leukemic cells.

Numerous methods are available in the art to determine whether any particular tumour cell expresses the class II, Class III and/or class IVb β-tubulin gene, including the use of commercially available tubulin isotype-specific antibodies and/or isotype-specific nucleic acid primers and/or probes.

The tumour cell when exposed to the nucleic acid construct may be *in vitro*, *in situ* in a tumour removed from a subject, or *in vivo.* The tumour cell will be of mammalian origin, and in one embodiment of human origin.

In one embodiment the nucleic acid construct sequences are introduced before commencement of treatment with the one or more microtubule destabilizing agents. The timing of the introduction of the nucleic acid construct sequences will depend on the route of administration of the construct and the kinetics of inhibition of the class II, class III and/or class IVb β-tubulin gene of the relevant target. The level of class II, class III and/or class IVb β-tubulin gene expression may be monitored for example using tissue biopsy specimens and real-time PCR specific for the class II, class III and/or class IVb β-tubulin gene expression. For siRNA administration to a cell, maximum gene suppression occurs at approximately 72 h after the siRNA has entered the cell, and this is the point that the cells are at maximum sensitivity to the one or more microtubule destabilizing agents. Accordingly preferably the administration of the microtubule destabilizing agent may be commenced at any time whereby the agent is present while the class II and/or class IVb β-tubulin gene expression of tumour cells is maximally suppressed.

Methods of treatment which involve the use of compositions comprising the nucleic acid constructs described herein and pharmaceutical compositions comprising the same are disclosed.

In general, suitable compositions for use in accordance with the disclosed methods and in accordance with the invention, may be prepared according to methods and procedures that are known to those of ordinary skill in the art and accordingly may include a pharmaceutically acceptable carrier, diluent and/or adjuvant.

Compositions may be administered by standard routes. In general, the compositions may be administered by the parenteral (e.g., intravenous, intraspinal, subcutaneous or intramuscular), oral or topical route. Administration may be systemic, regional or local. The particular route of administration to be used in any given circumstance will depend on a number of factors, including the nature of the condition to be treated, the severity and extent of the condition, the required dosage of the particular compound to be delivered and the potential side-effects of the compound.

In general, suitable compositions may be prepared according to methods which are known to those of ordinary skill in the art and may include a pharmaceutically acceptable diluent, adjuvant and/or excipient. The diluents, adjuvants and excipients must be "acceptable" in terms of being compatible with the other ingredients of the composition, and not deleterious to the recipient thereof.

In addition to the aforementioned agents which may increase the efficiency of transfection, examples of pharmaceutically acceptable carriers or diluents are demineralised or distilled water; saline solution; vegetable based oils such as peanut oil, safflower oil, olive oil, cottonseed oil, maize oil, sesame oils such as peanut oil, safflower oil, olive oil, cottonseed oil, maize oil, sesame oil, arachis oil or coconut oil; silicone oils, including polysiloxanes, such as methyl polysiloxane, phenyl polysiloxane and methylphenyl polysolpoxane; volatile silicones; mineral oils such as liquid paraffin, soft paraffin or squalane; cellulose derivatives such as methyl cellulose, ethyl cellulose, carboxymethylcellulose, sodium carboxymethylcellulose or hydroxypropylmethyl-cellulose; lower alkanols, for example ethanol or iso-propanol; lower aralkanols; lower polyalkylene glycols or lower alkylene glycols, for example polyethylene glycol, polypropylene glycol, ethylene glycol, propylene glycol, 1,3-butylene glycol or glycerin; fatty acid esters such as isopropyl palmitate, isopropyl myristate or ethyl oleate; polyvinylpyrridone; agar; carrageenan; gum tragacanth or gum acacia, and petroleum jelly. Typically, the carrier or carriers will form from 10% to 99.9% by weight of the compositions.

The compositions for use of the invention and of the disclosure may be in a form suitable for administration by injection, in the form of a formulation suitable for oral ingestion (such as capsules, tablets, caplets, elixirs, for example), in the form of an ointment, cream or lotion suitable for topical administration, in a form suitable for delivery as an eye drop, in an aerosol form suitable for administration by inhalation, such as by intranasal inhalation or oral inhalation, in a form suitable for parenteral administration, that is, subcutaneous, intramuscular or intravenous injection.

For administration as an injectable solution or suspension, non-toxic parenterally acceptable diluents or carriers can include, Ringer's solution, isotonic saline, phosphate buffered saline, ethanol and 1,2 propylene glycol.

Some examples of suitable carriers, diluents, excipients and adjuvants for oral use include peanut oil, liquid paraffin, sodium carboxymethylcellulose, methylcellulose, sodium alginate, gum acacia, gumtragacanth, dextrose, sucrose, sorbitol, mannitol, gelatine and lecithin. In addition these oral formulations may contain suitable flavouring and colourings agents. When used in capsule form the capsules may be coated with compounds such as glyceryl monostearate or glyceryl distearate which delay disintegration.

Adjuvants typically include emollients, emulsifiers, thickening agents, preservatives, bactericides and buffering agents.

Solid forms for oral administration may contain binders acceptable in human and veterinary pharmaceutical practice, sweeteners, disintegrating agents, diluents, flavourings, coating agents, preservatives, lubricants and/or time delay agents. Suitable binders include gum acacia, gelatine, corn starch, gum tragacanth, sodium alginate, carboxymethylcellulose or polyethylene glycol. Suitable sweeteners include sucrose, lactose, glucose, aspartame or saccharine. Suitable disintegrating agents include corn starch, methylcellulose, polyvinylpyrrolidone, guar gum, xanthan gum, bentonite, alginic acid or agar. Suitable diluents include lactose, sorbitol, mannitol, dextrose, kaolin, cellulose, calcium carbonate, calcium silicate or dicalcium phosphate. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

Liquid forms for oral administration may contain, in addition to the above agents, a liquid carrier. Suitable liquid carriers include water, oils such as olive oil, peanut oil, sesame oil, sunflower oil, safflower oil, arachis oil, coconut oil, liquid paraffin, ethylene glycol, propylene glycol, polyethylene glycol, ethanol, propanol, isopropanol, glycerol, fatty alcohols, triglycerides or mixtures thereof.

Suspensions for oral administration may further comprise dispersing agents and/or suspending agents. Suitable suspending agents include sodium carboxymethylcellulose, methylcellulose, hydroxypropylmethyl-cellulose, poly-vinyl-pyrrolidone, sodium alginate or acetyl alcohol. Suitable dispersing agents include lecithin, polyoxyethylene esters of fatty acids such as stearic acid, polyoxyethylene sorbitol mono- or di-oleate, -stearate or - laurate, polyoxyethylene sorbitan mono- or di-oleate, -stearate or -laurate and the like.

The emulsions for oral administration may further comprise one or more emulsifying agents. Suitable emulsifying agents include dispersing agents as exemplified above or natural gums such as guar gum, gum acacia or gum tragacanth.

Methods for preparing parenterally administrable compositions are apparent to those skilled in the art, and are described in more detail in, for example, Remington's Pharmaceutical Science, 15th ed., Mack Publishing Company, Easton, Pa.The topical formulations for use of the present invention or disclosure, comprise an active ingredient together with one or more acceptable carriers, and optionally any other therapeutic ingredients. Formulations suitable for topical administration include liquid or semi-liquid preparations suitable for penetration through the skin to the site of where treatment is required, such as liniments, lotions, creams, ointments or pastes, and drops suitable for administration to the eye, ear or nose.

The use of antisense oligonucleotide compositions for administration to the lungs, and compositions suitable for this use, are described for example in US patent serial no. 6,825,174. The methods described therein may be used to prepare suitable compositions for inhalable or intranasal delivery.

Drops for use according to the present invention, or of the disclosure, may comprise sterile aqueous or oily solutions or suspensions. These may be prepared by dissolving the active ingredient in an aqueous solution of a bactericidal and/or fungicidal agent and/or any other suitable preservative, and optionally including a surface active agent. The resulting solution may then be clarified by filtration, transferred to a suitable container and sterilised. Sterilisation may be achieved by filtration, followed by transfer to a container by an aseptic technique. Examples of bactericidal and fungicidal agents suitable for inclusion in the drops are phenylmercuric nitrate or acetate (0.002%), benzalkonium chloride (0.01 %) and chlorhexidine acetate (0.01%). Suitable solvents for the preparation of an oily solution include glycerol, diluted alcohol and propylene glycol.

Lotions for use according to the present invention, or of the disclosure, include those suitable for application to the skin or eye. An eye lotion may comprise a sterile aqueous solution optionally containing a bactericide and may be prepared by methods similar to those described above in relation to the preparation of drops. Lotions or liniments for application to the skin may also include an agent to hasten drying and to cool the skin, such as an alcohol or acetone, and/or a moisturiser such as glycerol, or oil such as castor oil or arachis oil.

Creams, ointments or pastes for use according to the present invention, or of the disclosure, are semi-solid formulations of the active ingredient for external application. They may be made by mixing the active ingredient in finely-divided or powdered form, alone or in solution or suspension in an aqueous or non-aqueous fluid, with a greasy or non-greasy basis. The basis may comprise hydrocarbons such as hard, soft or liquid paraffin, glycerol, beeswax, a metallic soap; a mucilage; an oil of natural origin such as almond, corn, arachis, castor or olive oil; wool fat or its derivatives, or a fatty acid such as stearic or oleic acid together with an alcohol such as propylene glycol or macrogols.

The composition may incorporate any suitable surfactant such as an anionic, cationic or non-ionic surfactant such as sorbitan esters or polyoxyethylene derivatives thereof. Suspending agents such as natural gums, cellulose derivatives or inorganic materials such as silicaceous silicas, and other ingredients such as lanolin, may also be included.

The composition may also be administered or delivered to target cells in the form of liposomes. Liposomes are generally derived from phospholipids or other lipid substances, and are formed by mono- or multi-lamellar hydrated liquid crystals that are dispersed in an aqueous medium. Specific examples of liposomes used in administering or delivering a composition to target cells are synthetic cholesterol (Sigma), the phospholipid 1,2-distearoyl-sn-glycero-3-phosphocholine (DSPC; Avanti Polar Lipids), the PEG lipid 3-N-[(-methoxy poly(ethylene glycol)2000)carbamoyl]-1,2-dimyrestyloxy-propylamine (PEG-cDMA), and the cationic lipid 1,2-di-o-octadecenyl-3-(N,N-dimethyl)aminopropane (DODMA) or 1,2-dilinoleyloxy-3-(N,N-dimethyl)aminopropane (DLinDMA) in the molar ratios 55:20:10:15 or 48:20:2:30, respectively, PEG-cDMA, DODMA and DLinDMA. Any non-toxic, physiologically acceptable and metabolisable lipid capable of forming liposomes can be used. The compositions in liposome form may contain stabilisers, preservatives, excipients and the like. The preferred lipids are the phospholipids and the phosphatidyl cholines (lecithins), both natural and synthetic. Methods to form liposomes are known in the art, and in relation to this specific reference is made to: Prescott, Ed., Methods in Cell Biology, Volume XIV, Academic Press, New York, N.Y. (1976), p. 33 et seq.

The use of DOTAP and/or other cationic lipid-mediated nucleic acid delivery systems is also contemplated. DOTAP has been used for the systemic delivery of a siRNA for gene slicing, for example (Sorenson et al., (2003) J. Mol. Biol. 327:761-766.

The composition may be conjugated to polycationic agents for the delivery of the composition into a cell. Polycationic agents can be used as delivery vehicles for compositions. One example of a polycationic agent is poly(ethyleneimine), or PEI. PEI is a low molecular weight compound which condenses short nucleic acid strands such as siRNA into positively charged particles which interaction with the anionic surface of a cell. PEI can be used alone or further conjugated to PEG. Another example of a polycationic agent is poly-L-lysine (PLL). Use of polycationic agents for the delivery of a composition to a cell is known in the art, and in relation to this specific reference is made to Judge et al. (2005). Nature 25: 457-462. PEI is present as a delivery vehicle according to the present invention.

The compositions may also be administered in the form of microparticles. Biodegradable microparticles formed from polylactide (PLA), polylactide-co-glycolide (PLGA), and epsilon-caprolactone (έ-caprolactone) have been extensively used as drug carriers to increase plasma half life and thereby prolong efficacy (Kumar, M., 2000, J Pharm Pharmaceut Sci. 3(2) 234-258). Microparticles have been formulated for the delivery of a range of drug candidates including vaccines, antibiotics, and DNA. Moreover, these formulations have been developed for various delivery routes including parenteral subcutaneous injection, intravenous injection and inhalation.

The compositions may comprise nanoparticles. Nanoparticles such as PEG-containing nanoparticles, have been demonstrated to readily taken up into cells because of their size. Where the compositions described herein are associated with nanoparticles, for example by complementary charges on the composition and the surface of the nanoparticles, the delivery of the composition into cells may be enhanced by the cell delivery properties provided by the nanoparticles.

The compositions may incorporate a controlled release matrix that is composed of sucrose acetate isobutyrate (SAIB) and organic solvent or organic solvents mixture. Polymer additives may be added to the vehicle as a release modifier to further increase the viscosity and slow down the release rate. SAIB is a well known food additive. It is a very hydrophobic, fully esterified sucrose derivative, at a nominal ratio of six isobutyrate to two acetate groups. As a mixed ester, SAIB does not crystallize but exists as a clear viscous liquid. Mixing SAIB with a pharmaceutically accepted organic solvent such as ethanol or benzyl alcohol decreases the viscosity of the mixture sufficiently to allow for injection. An active pharmaceutical ingredient may be added to the SAIB delivery vehicle to form SAIB solution or suspension formulations. When the formulation is injected subcutaneously, the solvent diffuses from the matrix allowing the SAIB-drug or SAIB-drug-polymer mixtures to set up as an in situ forming depot.

For the purposes of the present invention and disclosure molecules and agents may be administered to subjects as compositions either therapeutically or preventively. In a therapeutic application, compositions are administered to a patient already suffering from a disease, in an amount sufficient to cure or at least partially arrest the disease and its complications. The composition should provide a quantity of the molecule or agent sufficient to effectively treat the patient.

The therapeutically effective dose level for any particular patient will depend upon a variety of factors including: the disorder being treated and the severity of the disorder; activity of the molecule or agent employed; the composition employed; the age, body weight, general health, sex and diet of the patient; the time of administration; the route of administration; the rate of sequestration of the molecule or agent; the duration of the treatment; drugs used in combination or coincidental with the treatment, together with other related factors well known in medicine.

One skilled in the art would be able, by routine experimentation, to determine an effective, non-toxic amount of agent or compound which would be required to treat applicable diseases and conditions.

It will be understood that methods to determine whether the sensitivity of a tumour cell to a microtubule destabilizing agent has been enhanced may include the examination of any one or more of apoptotic markers, the degree of tumour shrinkage and the change in metabolic activity of tumour cells on exposure to a microtubule destabilizing agent. The presence of markers of apoptosis, including positive TUNEL labelling or the presence of DNA laddering may be used to determine whether cells in a tumour have undergone apoptosis.

The determination of changes in tumour size may be made using techniques readily available to the clinician, such as digital radiography, x-ray techniques including x-ray mammography, magnetic resonance imaging, computed tomography, positron emission tomography, gamma camera imaging, ultrasound imaging, endoscopic imaging and clinical assessment. The presence or level of tumour specific markers in the circulation or in a tissue biopsy may also be assessed using tumour specific antibodies or by in situ hybridization with tumour specific probes. Positron Emission Tomography may be used, for example, to measure the metabolic activity of tumours *in situ.*

### Examples

The examples are intended to serve to illustrate this invention.

Statistical analysis was done with GraphPad Prism program. Where statistical analysis is presented, results are expressed as means of at least three independent experiments ± SEM. A two-tailed Student's *t* test was used to determine the statistical differences between various experimental and control groups, with P<0.05 considered statistically significant.

### Example 1 - Cell culture and siRNA transfection

Human NSCLC cell lines Calu-6 and H460 (ATCC: Calu6 catalogue number HTB-56 and NCI-H460 catalogue number HTB-177 ) were maintained as monolayer cultures in Dulbecco's Modified Eagle Medium (DMEM) and RPMI, respectively, supplemented with 10% foetal calf serum (FCS) and 2 mM L-glutamine. The cells were grown at 37°C in a humidified atmosphere with 5% CO₂.

A variety of siRNA or shRNA were used in the examples described herein.

### Class III β-tubulin

(official symbol: TUBB3), also known as MCIR; TUBB4; beta-4

### SMARTpool, Human TUBB4, NM 006086 (class III β-tubulin)

Dharmacon RNA Technologies

### Class III β-tubulin Sequence 1

Sense sequence: GGGCGGAGCUGGUGGAUUCUU (SEQ ID NO:1)
   (Position: 327-345, mismatch at position 346-347)
Antisense sequence: 5Phos-GAAUCCACCAGCUCCGCCCUU (SEQ ID NO:2)

### Class III β-tubulin Sequence 2

Sense sequence: GUACGUGCCUCGAGCCAUUUU (SEQ ID NO:3)
   (Position 174-192)
Antisense sequence: 5Phos-AAUGGCUCGAGGCACGUACUU (SEQ ID NO:4)

### Class III β-tubulin Sequence 3

Sense sequence: GCGGCAACUACGUGGGCGAUU (SEQ ID NO:5)
   (Position 98-116)
Antisense sequence: 5Phos-UCGCCCACGUAGUUGCCGCUU (SEQ ID NO:6)

### Class III β-tubulin Sequence 4

Sense sequence: AGGAGUAUCCCGACCGCAUUU (SEQ ID NO:7)
   (Position 470-488)
Antisense sequence: 5Phos-AUGCGGUCGGGAUACUCCUUU (SEQ ID NO:8)

### Class III β-tubulin Sequence 5

Sense sequence: CAAGGUGCGUGAGGAGUAUUU (SEQ ID NO:9)
   (Position 459-477)
Antisense sequence: 5Phos-AUACUCCUCACGCACCUUGUU (SEQ ID NO:10)

Target sequence is the sense sequence, replacing U with T.

### 27-mer βIII siRNA sequences

Integrated DNA Technologies (IDT)

### 27-mer βIII siRNA Sequence-8

AGACAGAGACAGGAGCAGCUCACACGU (SEQ ID NO: 11)
5Phos-GUGUGAGCUGCUCCUGUCUCUGUCT (SEQ ID NO:12)
   (target sequence, replacing U with T; position 1521-1545)

### 27-mer βIII siRNA Sequence-11

UCUCACUCCAGCUGCGAGCAGCUUCAC (SEQ ID NO:13)
5Phos-GAAGCUGCUCGCAGCUGGAGUGAGA (SEQ ID NO:14)
   (target sequence, replacing U with T; position 1352-1376)

### βIII shRNA expression pRS vector

### Origene

(position 1521-1549).

### Class II β-tubulin

(Official symbol:TUBB2A), also known as TUBB; TUBB2; dJ40E16.7

### siGenoME ON-TARGETplus SMARTpool, Human TUBB2A, NM_001069

Dharmacon RNA Technologies

### Class II β-tubulin Sequence 7

Sense sequence: GCUGGUAACAAAUAUGUACUU (SEQ ID NO:16)
   (position 163-183; mismatch at position 182)
Antisense sequence: 5Phos-GUACAUAUUUGUUACCAGCUU (SEQ ID NO:17)

### Class II β-tubulin Sequence 8

Sense sequence: GAUCAAUCGUGCAUCCUUAUU (SEQ ID NO:18)
   (position 1350-1370; mismatch at position 1369)
Antisense sequence: 5Phos-UAAGGAUGCACGAUUGAUCUU (SEQ ID NO:19)

### Class II β-tubulin Sequence 9

Sense sequence: GAACUUCUGUUGUCCUCAAUU (SEQ ID NO:20)
   (position 1371-1389; mismatch at position 1390-1391)
Antisense sequence: 5Phos-UUGAGGACAACAGAAGUUCUU (SEQ ID NO:21)

### Class II β-tubulin Sequence 10

Sense sequence: GAAAUUCACACUGUUGAUGUU (SEQ ID NO:22)
   (position 1439-1458; mismatch at position 1459)
Antisense sequence: 5Phos-CAUCAACAGUGUGAAUUUCUU (SEQ ID NO:23)

Target sequence is the sense sequence, replacing U with T.

### Class IVb β-tubulin

(Official symbol: TUBB2C), also known as TUBB2

### siGenoME duplex, Human TUBB2, NM_006088

Dharmacon RNA Technologies.

### Class IVb β-tubulin Sequence 5

Sense sequence: GGGCAGUGCGGCAACCAAAUU (SEQ ID NO:24)
   (position 28-47; mismatch at 48)
Antisense sequence: 5Phos-UUUGGUUGCCGCACUGCCCUU (SEQ ID NO:25)

Target sequence is the sense sequence, replacing U with T.

For siRNA transfection, 3x10⁴-5x10⁴ cells/well were plated in 24-well plates and transfected with class II β-tubulin siRNA ON-Target plus SMARTpool reagent at up to 100nM siRNA (Dharmacon, Chicago, IL).

Non-silencing control siRNA, with no sequence homology to any known human gene sequence, was used as a negative control in all experiments (Qiagen, Valencia, CA), with the exception of 27-mer βIII siRNA experiments. Cells were transfected with βIII siRNA at a final concentration of 25 nM (Calu-6) and 100 nM (H460).

Transfection was carried out using Lipofectamine 2000 (Invitrogen, Carlsbad, CA) according to the manufacturer's instructions. Control experiments were done in parallel by transfecting the cells with a non-silencing control siRNA with no sequence homology to any known human gene sequence (Qiagen, Valencia, CA) at equivalent concentrations as the target siRNA. Cells were harvested for experiments after 48-72h transfection, unless otherwise stated. All experiments involved the use of a Lipofectamine only control (mock-transfected) and a non-silencing control siRNA (negative control).

### Example 2 - Analysis of β-tubulin isotypes

The effect of siRNA transcription on the expression of β-tubulin isotypes was assessed using reverse transcription-PCR (RT-PCR) analysis of β-tubulin isotypes and by western blotting. For reverse transcription analysis, total RNA was isolated using Trizol reagent (Invitrogen), according to the manufacturer's instructions. RNA samples were DNAse-treated, and reverse transcribed for RT-PCR analysis using methodology and specific primers as described in Kavallaris et al. (1997) J Clin Invest 100, 1282-1293.For Hβ9 (class II), H5β (class IVa) and Hβ2 (class IVb) genes, semi-quantitative PCR-based assays involved setting up two separate PCR tubes for target (β-tubulin) and control (β₂-microglobulin) gene sequences. Amplified products were resolved on a 12.5% polyacrylamide gel and visualized by ethidium bromide staining using the Gel Doc 1000 imaging system with data analysis using QuantityOne software version 4.0 (Bio-Rad, Hercules, CA). PCR amplifications were performed in triplicate using two independent cDNA preparations.

For western blotting, total cellular proteins from transfected cells (10 µg) were resolved on 12% SDS-PAGE and electrotransferred to nitrocellulose membrane using standard methods. Immunoblotting was performed using monoclonal antibodies to class I β-tubulin (1:5,000; kindly provided by Dr. R. Luduena, University of Texas, San Antonio, TX) or alternatively Anti β-I tubulin (AbCam), class II β-tubulin (1:1,000, Covance, Richmond, CA), class III β-tubulin (1:1,000, Covance), class IV β-tubulin (1:500, Sigma Chemical Co.-Aldrich, St Louis, MO) and total β-tubulin (1:500, Sigma) as described previously (Don et al. (2004) Mol Cancer Ther 3, 1137-1146) with minor modifications. Enhanced chemiluminescence (GE Healthcare, Uppsala, Sweden) was used for detection. The blots were scanned using the typhoon scanner and quantified using ImageQuant software version 5.2 (Molecular Dynamics Inc, Sunnyvale, CA). The experiments were repeated in triplicate with proteins isolated from three independent extractions.

As shown in Figure 1A, when treated with the optimal amount of siRNA, βII tubulin (Hβ9) expression was greatly reduced in both cell lines at the gene level, with complete suppression of βIVb (Hβ2) expression observed in class IVb siRNA-transfected cells. At 72 h, western blot analysis indicated that levels of class II β-tubulin and class IV β-tubulin were markedly reduced in cells transfected with siRNA for βII or βIVb tubulin, respectively, but not in control siRNA (Fig. 1B). The specificity of the siRNAs was proven by probing with specific antibodies of other β-tubulin isotypes. As shown in Fig. 2, downregulation of either class II (Fig. 2A) or class IVb β-tubulin (Fig. 2B) did not affect the endogenous levels of other β-tubulin isotypes.

### Example 3 - Clonogenic assay of drug resistance

Cells were transfected for 24 h and approximately 600 cells (for Calu-6) or 150 cells (for H460) were seeded into each well of a 6-well plate and allowed to attach for 4-6 h. The cell lines used in this study were not subject to prior drug selection, and are likely to represent intrinsic drug resistance observed in lung carcinoma.

Cells were then treated with increasing concentrations of various anti-mitotic drugs. Following a 3-day incubation at 37°C, the drug-containing medium was removed and replaced with fresh complete medium. Medium was changed at 3-4 day intervals for 7 to 10 days until visible colonies formed. Control cells were treated identically, with comparable media changes. Surviving colonies were simultaneously fixed and stained with 0.5% crystal violet in methanol, rinsed with water to remove excess stain and air-dried overnight.

The colonies in each well were manually counted, and the results were presented as surviving fraction according to the formula: surviving fraction was calculated as follows: colony number/ (number of cells seeded x plating efficiency), where plating efficiency is equivalent to the colony number divided by the number of cells seeded in the drug-free medium. Dose-response curves were plotted for each drug-cell line combination and inhibitory dose (ID₅₀) values were extrapolated from this curve using GraphPad Prism program (GraphPad Software, version 4, San Diego, CA). ID₅₀ was defined as the concentration of the drug required to reduce the number of colonies in drug-treated wells to 50% of that of the relevant untreated control wells. The ID₅₀ values were the means of at least four independent experiments.

The results of this assay demonstrated that the knockdown of βII and βIVb tubulin significantly enhanced the cytotoxicity of vinca alkaloids but not of taxanes. As shown in Fig. 3A-B, knockdown of βII significantly sensitized both cell lines to vincristine but not paclitaxel. Surprisingly, the downregulation of βIVb tubulin led to a precipitous drop in surviving colonies when treated with vincristine (Fig. 4A and Fig. 5). Similar to the observations of class II transfectants, the downregulation of βIVb did not significantly enhance the cytotoxicity of paclitaxel in these cells (Fig 4B).

To rule out the possibility that these effects might be due to some unique interaction of paclitaxel with specific β-tubulin isotypes, an additional microtubule-stabilizing agent, epothilone B was included in the analysis. Treatment with epothilone B led to no significant change in drug sensitivity in H460 cells transfected with βII siRNA (Fig. 3C), consistent with the paclitaxel data. In contrast, class IVb transfectants were significantly more resistant to epothilone B (Fig. 4C). Additional vinca alkaloids were also tested to ascertain the hypersensitivity effects observed on vincristine in both class II and IVb transfectants. The results of these assays are shown in Table 1A for βII siRNA and Table 1B for βIVb siRNA. The results obtained for all of the vinca alkaloids examined were consistent in both NSCLC cell lines, although only the H460 cell data is presented.

Although it was possible to specifically distinguish class IVa (H5β) and class IVb (Hβ2) expression at the gene expression (mRNA) level, there are currently no commercially available antibodies which differentiate between class IVa and class IVb protein, and so the differential expression of these polypeptides was not examined in this study. A combination of affinity capture with class IV β-tubulin specific antibodies and high resolution electrophoresis techniques, such as capillary zone electrophoretic techniquesis expected to resolve these two proteins and allow their differential quantification.

A reduction of approximately 40-50% H5β gene expression in Calu-6 cells transfected with class IVb siRNA was observed (data not shown) but no significant changes were observed in H460 class IVb transfectants. Since both cell lines showed identical clonogenic results, the drug response obtained was attributed to class IVb suppression rather than class IVa.

Inhibition of either βII or βIVb in NSCLC cells did not significantly potentiate the cytotoxicity of paclitaxel. This result is consistent with the observation that transfection of CHO cells with β-tubulin classes I, II or IVb did not confer resistance to paclitaxel.

The siRNA used in this study resulted in the specific downregulation of expression of each targeted β-tubulin isotype, and no change was observed in the levels of the other β-tubulin isotypes. These results highlight the differing contribution of each individual β-tubulin isotype in determining the chemosensitivity of a cell.

Based on these results, we propose that each β-tubulin isotype is unique in terms of drug interaction, and that the isotype composition of a cell affects its response to antimicrotubule agents. The dramatic effects on vincristine sensitivity observed in βIVb transfectants is of interest, since this isotype is constitutively expressed in all tissues.

It is conceivable that differences in β-tubulin composition may influence the interactions of microtubules with other elements of the cytoskeleton network and hence dictate tumour cell behaviour, including the cellular response to chemotherapeutic agents. Another possible explanation for the mechanism of sensitivity is alterations in drug binding affinity; however, since vinca alkaloids (including vincristine, vinblastine and vinorelbine) all bind to β-tubulin isotypes with similar affinity, it is unlikely that alterations in drug binding affinity due to the different isotype composition is the underlying mechanism.

### Example 4 - Immunofluorescent staining of tubulin

Transfected Calu-6 cells were grown on sterile chamber slides until 60-70% confluent. Cells were then treated with paclitaxel and vincristine for 1h at 10 nM. Cells were fixed in methanol and processed for staining as previously described (Don *et al.* (2004) *supra*) with minor modifications. Fixed cells were stained with α-tubulin (1:400 in 5% FCS/PBS, Sigma) for 30 min followed by Cy2 anti- mouse fluorescent tagged antibody (1:1,000 in 5% FCS/PBS, GE Healthcare). Slides were mounted on a coverslip using DAPI II Counterstain (Vysis Inc., Downers Grove, IL). Slides were viewed using a Zeiss Axioplan 2 Immunofluorescence Microscope (Mannheim, Germany) with Image-Pro Plus 4.1 software (Media Cybernetics, L.P., Silver Spring, MD).

As shown in Fig. 7A, both untreated βII and βIVb transfectants showed no observable changes to microtubule morphology. However, after incubation with 10 nM vincristine, both βII and βIVb siRNA-transfected cells showed a dramatic disruption of the microtubule cytoskeleton when compared to the control siRNA. In the βII and βIVb siRNA-transfected cells microtubules were largely depolymerized and most of the cells were rounded with occasional small remnants of microtubules remaining. In contrast, the morphology of control cells was less affected by vincristine. Although the morphology of microtubules was unchanged in both βII and βIVb transfectants in the absence of vincristine, low concentrations of vincristine caused extensive microtubule disruption in these cells compared to the controls.

The effects of paclitaxel treatment on βII transfectants was also examined. βII siRNA-transfected cells were stained with α-tubulin after treated with 10 nM of paclitaxel for 1 h. Consistent with the clonogenic data, microtubules of βII siRNA-treated cells did not appear significantly different from those treated control cells (Fig. 7B).

### Example 5 - Examination of Intracellular accumulation of vincristine

To determine whether changes in drug accumulation were contributing to drug hypersensitivity, the accumulation of [³H]-vincristine was measured in βIVb siRNA-transfected cells and MRP1-positive MCF7-VP16 cells as a positive control.

Intracellular vincristine accumulation was quantitated using radio labelled drug as previously described (Verrills, N. M., Flemming, C. L., Liu, M., Ivery, M. T., Cobon, G. S., Norris, M. D., Haber, M., and Kavallaris, M. (2003) Chem Biol 10, 597-607) with minor modifications. Briefly, transfected cells were incubated at 37°C for 2 h in the presence of [³H]-vincristine (5.20 Ci/mmol; final concentration, 50nmol) (Moravek Biochemicals Inc, California). Cells were washed, hydrolyzed and the [³H] radioactivity counted as previously described. An aliquot of cell lysate was used in parallel to determine the cellular protein concentration using the BCA assay kit (Pierce, Rockford, IL). Intracellular vincristine accumulation was determined for duplicate samples and expressed as pmoles of vincristine/mg of protein. MCF7-VP16 cells (Schneider et al. Cancer Res. (1994) 54(1):152-8) were included as a positive control, as these cells had previously been shown to overexpress multidrug-resistance associated protein-1 (MRP1).

MCF7-VP16 cells showed a significant decrease in the accumulation of [³H]-vincristine (data not shown). There was no significant difference in intracellular [³H]-vincristine levels between βIVb knockdown and controls in both Calu-6 and H460 (Fig. 6). These results suggest that enhanced sensitivity of vincristine in βIVb knockdown is not attributable to an increase in drug accumulation.

### Example 6 - Cell cycle analysis

The distribution of DNA content in βII or βIVb tubulin siRNA-transfected H460 cells was determined by flow cytometry. After 72h transfection, transfected cells were exposed to vincristine at 5nM or 40nM concentrations for 24h. On the day of analysis, both adherent and floating cells were harvested, washed with PBS and then stained with a solution containing 0.4% Triton X-100 (Sigma), 50 µg/ml propidium iodide (Sigma), and 2 µg/ml DNase-free RNase (Roche, Indianapolis, IN) in the dark for 15 min at 37°C. DNA content was measured by a FACSCalibur flow cytometer (Becton Dickinson, San Diego, CA). The flow rate was <200 nuclei per second and 10,000 cells from each sample were analysed. Measurements were performed under the same instrumental settings. The CellQuest program was used to quantitate the distribution of cells in each cell cycle phase: sub-G₁ (apoptotic cells), G₁, S and G₂/M.

As shown in Figures 8A and 8B, the inhibition of βII or βIVb tubulin alone did not significantly affect the cell cycle profiles of untreated H460 cells in comparison with the controls. However, upon treatment with 5nM vincristine, both βII and βIVb transfectants showed an increase in the G₂/M and sub-G₁ populations. Class II transfectants showed a greater G₂/M accumulation when treated with 40 nM vincristine, similar to the control-treated cells, with no significant difference in the sub-G₁ population (Fig. 8A).

Class IVb transfectants, on the other hand, failed to undergo G₂/M arrest and demonstrated a concomitant increase in the G₀-G₁ population when treated with equivalent concentrations of vincristine with (Figure 8B). Although mitotic block was not apparent, the cells nevertheless exhibited apoptosis as shown by an increase in the sub-G₁ populations.

### Example 7 - Cytotoxic drugs for βIII-tubulin knockdown studies

The examples that follow describe studies in which the role of βIII tubulin knockdown on the sensitivity of tumour cells was investigated.

Paclitaxel (Calbiochem, Merck Biosciences, Nottingham, U.K.) was prepared at a stock concentration of 2 mM in dimethyl sulfoxide (DMSO). Vincristine (Sigma-Aldrich, St Louis, MO) was prepared at a stock concentration of 2 mM in saline (0.9% wt/vol NaCl); vinorelbine (kindly provided by Dr B Hill, Pierre Fabre, France) was solubilized in water at a stock concentration of 2 mM. Doxorubucin (doxorubicin hydrochloride; Pfizer, Sydney, Australia) was prepared at a stock concentration of 3.45 mM in saline. Etoposide (VP-16; Sigma-Aldrich) was prepared at a stock concentration of 68 mM in DMSO and cisplatin (Pharmacia, Rydalmere, Australia) was prepared at a stock concentration of 3.3 mM in saline.

### Example 8 - Silencing of βIII-tubulin in NSCLC cell lines

The effects of βIII-tubulin siRNA transfection on the gene and protein expression of the isotype, were assessed by RT-PCR and western blotting. For RT-PCR, total RNA was extracted from the transfected cells using the Trizol reagent (Invitrogen), according to the manufacturer's instructions. RNA samples were DNAse-treated, reverse transcribed for RT-PCR analysis using methodology and specific primers as described in detail previously (Kavallaris et al. (1997) J Clin Invest 100, 1282-1293). Beta-2-microglobulin β₂M) was used as an internal mRNA control. Preparation of protein lysates and Western blot analysis were performed as follows. Briefly, total cellular proteins were separated on 4% to 15% SDS-PAGE and electrotransferred to nitrocellulose membranes using standard methods. Immunoblotting was carried out using monoclonal antibodies directed against βI-tubulin (clone SAP 4G5, Abcam Ltd., Cambridgeshire, UK), βII-tubulin (clone 7B9, Chemicon, Temecula, CA), βIII-tubulin (clone TUJ1, Chemicon), and βIV-tubulin (clone ONS 1A6, Sigma-Aldrich), GAPDH (Abcam Ltd.). Primary antibodies were detected using horseradish peroxidase-linked secondary antibody (Amersham Pharmacia Biotech, Uppsala, Sweden) and enhanced chemiluminescence (GE Healthcare, Uppsala, Sweden) was used for detection. The blots were scanned using the Typhoon^{®} scanner and quantified using ImageQuant software version 5.2 (Molecular Dynamics Inc, Sunnyvale, CA).

Treatment of H460 and Calu-6 cells with βIII-tubulin siRNA resulted in significant knockdown of βIII-tubulin mRNA levels compared to the mock- and control siRNA-transfected cells (Fig. 9A). This result was consistent with the decrease observed at the protein level (Fig. 9B). The βIII-tubulin siRNA specifically targeted βIII-tubulin and had no cross-reactivity with other β-tubulin isotypes examined as demonstrated by western blotting (Fig. 9C). In addition, silencing of βIII-tubulin did not cause compensatory changes in the other isotypes examined.

### Example 9 - Silencing of class III β-tubulin disrupts microtubules upon paclitaxel or vincristine treatment

To assess the effects of βIII downregulation on microtubule organization, immunofluorescence staining was performed on transfected Calu-6 cells as follows. Briefly, siRNA transfected cells were plated in glass chamber slides and allowed to reach 70% confluence. Cells were then treated with 10 nM of paclitaxel or vincristine for 1 hour. Drug was then removed by washing cells with PBS and blocked in 10% FCS/PBS. For dual staining, cells were first labelled with an antibody to class III β-tubulin wich was exposed to cells incubated at 37°C for 30 minutes in a humidified chamber. Following washes in 0.1% Tween 20/PBS at room temperature, the slides were incubated with Cy3 anti-mouse fluorescent-tagged antibody (GE Healthcare) for 40 minutes in a humidified chamber in the dark at room temperature. Following incubation, the slides were washed again in 0.1% Tween 20/PBS. This was then followed by staining with α-tubulin and Cy2 anti-mouse fluorescent tagged antibody (GE Healthcare). Slides were mounted on a coverslip using DAPI II Counterstain (Vysis Inc., Downers Grove, IL). Immunofluorescence microscopy was performed and images were captured using a Sensicam Charged Coupled Device camera (PCO Imaging, Kelheim, Bavaria, Germany) and the Image-Pro Plus 4.1 software (Media Cybernetics, L.P., Silver Spring, MD).

The βIII siRNA-transfected cells showed no observable changes to microtubule morphology. Consistent with the Western blot data, a clear decrease in class III β-tubulin immunofluorescence intensity was observed in the βIII-tubulin siRNA treated cells compared to both the control siRNA- and mock-transfected cells when imaged under identical conditions. Control cells (mock- and control siRNA-transfected cells) expressed similar levels of class III β-tubulin as compared to βIII knockdown where βIII-tubulin expression levels were almost undetectable by fluorescence microscopy.

To examine the effect of tubulin-binding agents on βIII-tubulin siRNA treated cells, cells were exposed for 1 h to either 10 nM paclitaxel or vincristine and cellular morphology was examined. As shown in Fig. 10, βIII siRNA-transfected Calu-6 cells showed extensive disruption of the microtubule cytoskeleton as compared to the control treated cells. The majority of the βIII siRNA treated cells were rounded and many of these cells displayed abnormal cellular or nuclear morphology (arrows, Fig. 10). The control treated cells occasionally showed minimal bundling of microtubules normally associated with paclitaxel treatment but the frequency of rounded cells and the extent of microtubule disruption was minimal compared to βIII transfectants.

### Example 10 - βIII-tubulin silencing increases sensitivity to Tubulin-binding agents and DNA damaging agents

The data in the preceding example suggested that silencing βIII-tubulin expression may have increased the sensitivity of tumour cells to both paclitaxel and vincristine. To quantitate any change in drug sensitivity, drug treated clonogenic assays were performed. Twenty-four hours after siRNA transfection, cells were harvested and plated into 6-well plates for 6 h, prior to the addition of various drugs as indicated in the figure legends. After 72 h incubation, the drug-containing media was removed and replaced with complete growth media. Medium was changed every 3 days for 7 to 10 days until visible colonies formed. Colonies were simultaneously fixed and stained with 0.5% crystal violet in methanol, and manually counted. Individual stained colonies in each well were counted and the surviving fraction was calculated as follows: colony number/(number of cells seeded x plating efficiency), where plating efficiency equals the colony number divided by the number of cells seeded in drug-free media.

Cellular uptake and retention of tritiated substrate [³H]-paclitaxel and ³H]-vincristine were measured as previously described in Verrills, et al. J Natl Cancer Inst 2006; 98:1363-74. Briefly, cells were transfected in 12-well plates for 48 h. Drug uptake was monitored by adding [³H]-paclitaxel (14.7Ci/mmol; final concentration, 50 nM; Moravek Biochemicals Inc, Brea, CA) or [³H]-vincristine (7.1 Ci/mmol; final concentration of 12.5 nM; GE Healthcare) to the transfected cells for 2 h at 37°C. Cells were washed, hydrolyzed and counted as described in previous examples. The amount of tritiated drug accumulated in the cells was determined for duplicate samples and expressed as pmoles of the drug/mg protein. At least three independent experiments were performed. Relevant positive controls were included, including vincristine resistant neuroblastoma (BE/VCR10) and VP-16 resistant breast cancer cells (MCF7-VP16), which are known to overexpress multidrug resistance 1 (MDR1) and multidrug resistance associated protein 1 (MRP 1) respectively.

Consistent with the immunofluorescence observations, βIII-tubulin silencing resulted in a significant increase in sensitivity to paclitaxel and vincristine (Fig. 11A and B). In addition, the βIII-tubulin silenced cells exhibited enhanced sensitivity to vinorelbine compared to controls (Figure 11D). The increased sensitivity was not due to altered accumulation of drug as there was no significant difference in the intracellular drug accumulation levels in the βIII-tubulin siRNA treated Calu-6 or H460 NSCLC cells compared to mock and control siRNA treated cells (data not shown).

Drug treated clonogenic assays were also performed using DNA damaging agents, VP-16, cisplatin and doxorubicin. Interestingly, βIII-tubulin silencing resulted in increased sensitivity to all three DNA damaging agents tested in H460 cells (Fig. 11C). Similar results were obtained with Calu-6 cells. Since H460 cells have wild-type p53 and the Calu-6 cells harbour mutated p53, sensitivity to these drugs appears to be independent of the p53 genotype.

### Example 11 - Knockdown of βIII-tubulin abrogates paclitaxel- and vincristine-induced G2/M arrest and induces an increase in sub-G1 population

To determine whether βIII-tubulin silencing affects the cell cycle profiles, cell cycle analysis using flow cytometry was performed. Cell cycle analysis was determined by transfecting H460 cells with siRNA for 72 h and harvesting (adherent and suspension) cells 24 h after drug treatment. DNA content was stained for 15 min at 37°C with a solution containing 0.4% Triton X-100 (Sigma-Aldrich), 50 µg/ml propidium iodide (Sigma-Aldrich), and 2 µg/ml DNAse-free RNAse (Roche, Indianapolis, IN). The cells were then analyzed for cell cycle perturbation using a FACSCalibur (Becton-Dickinson, Franklin Lakes, NJ). The CellQuest program was used to quantitate the distribution of cells in each cell cycle phase: sub-G₁ (apoptotic cells), G₁, S and G₂/M.

The cell cycle profiles of H460 cells were not affected by βIII-tubulin silencing. To determine whether the tubulin-binding agents affected the cell cycle profiles of βIII-tubulin siRNA transfectants, cells were treated with either paclitaxel or vincristine. Following 24 h incubation with 5 nM paclitaxel, the βIII-tubulin silenced cells had a higher sub-G₁ (apoptotic cells) content compared to the control siRNA treated cells (p<0.05) (Fig. 12A), although both the βIII-tubulin siRNA and control siRNA treated cells had a similar increase in G₂/M content compared to untreated samples. A major difference was observed with 40 nM paclitaxel, with the control siRNA treated cells showing a marked G₂/M block while the βIII-tubulin siRNA treated cells had a marked increase in the sub-G₁ population reflective of apoptotic cells (Fig. 12A). Similar results were observed when siRNA treated cells were exposed to vincristine (Fig. 12B), suggesting that there is a common mechanism that enhances the effects of both the taxanes and *vinca* alkaloids following βIII-tubulin silencing.

### Example 12 - Knockdown of βIII-tubulin increases sensitivity of cells to apoptosis in the presence of either paclitaxel or cisplatin

Annexin V-FITC staining followed by flow cytometry analysis was performed to address whether the increase in the sub-G₁ population following exposure to tubulin-binding agents in the βIII-tubulin siRNA treated cells was related to an increase in apoptosis induction. Apoptosis induction was determined by transfecting H460 cells with siRNA for 72 h and harvesting (adherent and suspension) cells 48 h after drug treatment, as previously described in Pasquier et al., Mol Cancer Ther 2004; 3:1301-10. Briefly, 1x10⁵ cells were incubated with Annexin V-FITC and propidium iodide for 15 min in the dark (Becton-Dickinson), immediately followed by flow cytometry using a FACSCalibur (Becton-Dickinson). Cytogram analysis was performed using Cell Quest software.

Incubation of H460 cells with paclitaxel for 48 h, induced apoptosis from 1 nM in βIII-tubulin siRNA treated cells and from 5 nM in control siRNA treated cells (Fig. 13A). Moreover, at all tested paclitaxel concentrations (1, 2 and 5 nM), the percentage of apoptotic cells was significantly higher in βIII-tubulin siRNA treated cells than in control siRNA treated cells (Fig. 13A).

Similarly, in βIII-tubulin siRNA treated cells exposed to cisplatin for 48 h, there was a significant increase in the number of apoptotic cells in the 0.4 or 1 µM cisplatin treated cells compared to controls (Fig 13B). Taken together, this data demonstrates that βIII-tubulin silencing sensitized cells to apoptosis induction following tubulin-binding agent and DNA damaging agent treatment in NSCLC.

### Example 13 - Class III β-tubulin silencing increases sensitivity to the Tubulin-stabilizing agent epothilone

Class III β-tubulin knockdown of H460 and Calu-6 cells was carried out as described in previous examples, and drug treated clonogenic assays were performed using the tubulin stabilizing agent epothilone B. The results of these experiments are illustrated in Figure 14.

Knockdown of class III β-tubulin in both cell lines increased the sensitivity of these cells to epothilone B.

### Example 14 - Alternative siRNAs for Class III β-tubulin silencing

In order to investigate whether knockdown of tubulin expression by other siRNA was able to induce equivalent levels of class III β-tubulin silencing changes to responses to tubulin-binding agents, class III β-tubulin expression in H460 cells was knocked down using 27mer-siRNA reagents designated sequence 8 (SEQ ID NOS: 11 and 12) or sequence 11 (SEQ ID NOS: 13 and 14). These siRNAs recognise different regions of the class III β-tubulin mRNA sequence.

The results of these experiments are illustrated in Figures 15 and 16 and in Table 2. Western blots illustrated in Figure 15 demonstrate that each of these siRNAs was effective in knocking down class III β-tubulin protein expression to varying extents, while not influencing the expression of other tubulin isotypes. Figure 16 and Table 2 demonstrate that the knocked down cells also exhibited varying degrees of increased sensitivity to both paclitaxel and vincristine, suggesting that a range of siRNAs may be used to successfully enhance the sensitivity of tumour cells to a tubulin-binding agent.

### Example 15 - Short Hairpin RNA (shRNA) for Class III β-tubulin silencing

A short hairpin RNA construct (SEQ ID NO:15) was used in an attempt to stably knock down the expression of class III β-tubulin in H460 cells.

Three clones, designated clone 4, clone 59 and clone 60, were identified in which stable class III β-tubulin knock down was observed. As illustrated in Figure 17, each of these clones demonstrated a different level of knockdown of the class III β-tubulin isotype (as illustrated by the variation in intensity of the class III β-tubulin protein detected in normalised Western blots), without apparently altering the expression of other tubulin isotypes. When these stably knocked down clones were examined for sensitivity to paclitaxel or the DNA-damaging agent cisplatin (CDDP) in a clonogenic assay (Figure 18 and Table 3), all clones were found to exhibit increased sensitivity to each of these agents. The sensitivity of each of the clones appeared to correlate with the degree of knockdown of class III β-tubulin, with clone 4 appearing to have both the greatest amount of knockdown and the greatest sensitivity to each of the agents.

### Example 16 - 2 methoxyestradiol resistance in leukemic cells is associated with class II β-tubulin

The tubulin-binding agent 2-methoxyestradiol (2ME2) binds to β-tubulin near the colchicine-binding site, inhibits microtubule polymerization, and induces mitotic arrest. CCRF-CEM Leukaemia cells that display increasing resistance to 2ME2 were selected, and four of the highly 2ME2 resistant leukaemia sublines were chosen for detailed analysis. The 2ME2 cells selected in 3.6-28.8µM 2ME2 were found to be 11- to 107-fold more resistant to 2ME2 than cells of the parent line.

The 2ME2-resistant cells were hypersensitive to epothilone B and only the lowest resistant subline, CEM/2ME2-3.6R, was cross-resistance to colchicine and vincristine. The 2ME2-resistant cells did not exhibit G2/M cell cycle arrest as demonstrated in the parental cells and demonstrated higher levels of polymerized tubulin than the parent cells. Four class I β-tubulin mutations, S25N, D197N, A248T and L350N, were detected in the 2ME2-resistant cells. The S25N mutation is within the paclitaxel-binding site, whereas A248T and L350N are within colchicine-binding site on β-tubulin, yet the resistant cells were not cross resistant to paclitaxel or colchicine.

This suggests that the mutations may have induced conformational change to the binding sites. In addition, 2ME2 resistant sublines were demonstrated to have upregulated their expression of class II β-tubulin (Figure 19).

To determine whether the levels of class II β-tubulin could predict sensitivity and be contributing to resistance, siRNA mediated knockdown of class II β-tubulin in drug sensitive H460 (NSCLC) cells was performed. Knockdown of class II β-tubulin significantly increased sensitivity of H460 cells to 2ME2 and colchicine.

### Example 17 - Administration of nucleic acid construct to a subject

A subject diagnosed with or suspected of having a NSCLC tumour undergoes a tumour biopsy. Biopsy tissue is probed with specific commercially available monoclonal antibodies or nucleic acids by RT-PCR as described herein or as previously described (Kavallaris et al. (1997) J Clin Invest 100:1282) to determine whether the tumour expresses βII, βIII or βIVb β-tubulin mRNA or protein. If the tumour expresses βII, βIII or βIVb β-tubulin the subject is treated according to the method disclosed herein.

One or more siRNA constructs directed to class II, class III or class IVb β-tubulin, either encapsulated in a cationic liposome as described previously (Nogawa *et al.* (2005) *supra*)*,* conjugated with a nanoparticle or suspended in saline, and optionally synthesised using nuclease-resistant chemical modifications, is injected directly into the tumour at a dose of 2 x 10⁹ constructs/injection, and the injections repeated every second day 3 or 5 times. Alternatively the construct conjugated to a nanoparticle is administered systemically, where the ability of the nanoparticle to localise the construct is the lungs is utilised.

In order to monitor the inhibition of class II, class III or class IVb β-tubulin expression in the tumour, a further biopsy may be taken at approximately 72 hours and the tissue examined for gene expression as described above.

After substantial inhibition of expression of class II, class III or class IVb β-tubulin in the tumour, the subject is administered the standard dose of tubule-binding agent for the tumour type in question using the standard rate of administration.

**Table 1A. Clonogenic survival of βII siRNA-transfected NSCLC H460 cells in the presence of vinca alkaloids**

| Transfection | Vinblastine (nM) | | Vinflunine(nM) | | Vinorelbine (nM) | |
|---|---|---|---|---|---|---|
| | ID₅₀* | *P*† | ID₅₀ | *P*† | ID₅₀ | *P*† |
| Mock | 0.658 | | 30.990 | | 6.344 | |
| Control siRNA | 0.749 | 0.39 | 29.859 | 0.67 | 5.780 | 0.37 |
| βII siRNA | 0.437 | .004 | 18.976 | <.0001 | 4.036 | .005 |

| | | | | | | |
|---|---|---|---|---|---|---|
| *ID₅₀ = concentration of drug that killed 50% of the cells (ID₅₀); † Student's *t* test (two-sided). *P* value was determined by comparing the ID₅₀ of the mock-transfected cells with the ID₅₀ of the siRNA-transfected cells. | | | | | | |

**Table 1B. Clonogenic survival of βIVb siRNA-transfected NSCLC H460 cells in the presence of vinca alkaloids**

| Transfection | Vinblastine (nM) | | Vinflunine(nM) | | Vinorelbine (nM) | |
|---|---|---|---|---|---|---|
| | ID₅₀* | *P*† | ID₅₀ | *P*† | ID₅₀ | *P*† |
| Mock | 0.674 | | 31.684 | | 6.950 | |
| Control siRNA | 0.615 | 0.49 | 30.483 | 0.25 | 6.516 | 0.39 |
| βIVb siRNA | 0.173 | <.0001 | 19.177 | <.0001 | 5.431 | .0008 |

**Table 2**

| H460 | Paclitaxel (nM) | VCR (nM) |
|---|---|---|
| | ID₅₀¹ ± SEM (RS², *P-value*) | ID₅₀¹± SEM (RS², *P-value*) |
| **Mock** | 2.628±0.136 | 3.989±0.158 |
| **Ctrl-1** | 2.891±0.165 (0.91, *NS*) | 4.246±0.180 (0.94, *NS*) |
| **27-mer βIII siRNA Sequence**-**8** | 1.699±0.043 (1.55, <*0.0001****) | 1.683±0.116 (2.37, <*0.0001****) |
| **27-mer βIII siRNA Sequence-11** | 1.781±0.070 (1.42, 0.0001***) | 2.947±0.175 (1.34, 0.001**) |

Drug treated clonogenic assay on H460 cells following βIII silencing using 27-mer βIII siRNA

**Table 3**

| H460 | Paclitaxel (nM) | CDDP (µM) |
|---|---|---|
| | ID₅₀¹ ± SEM (RS², *P-value*) | ID₅₀¹ ± SEM (RS², *P-value*) |
| **Ctrl clone 1** | 2.755±0.131 | 0.407±0.039 |
| **Ctrl clone 2** | 2.829±0.093 (0.97, *NS*) | 0.383±0.041 (1.06, *NS*) |
| **βIII shRNA clone 4** | 1.359±0.087 (2.03, <*0.0001****) | 0.169±0.012 (2.42, *0.0002****) |
| **βIII shRNA clone 59** | 1.781±0.070 (1.55, <0.0001***) | 0.219±0.013 (1.86, 0.001**) |
| **βIII shRNA clone 60** | 1.583±0.095 (1.74, <0.0001***) | 0.237±0.012 (1.72, 0.0019**) |

| | | |
|---|---|---|
| Drug treated clonogenic assays of H460 βIII-shRNA stable cells ¹Drug concentration required to inhibit 50% of colony formation. ²Relative sensitivity (RS) is the fold sensitivity of the siRNA treated cells compared to ctrl clone 1. RS was determined by dividing the ID₅₀ of ctrl clone 1 by the ID₅₀ of the βIII stable clones or ctrl clone 2. *NS -* not significant | | |

## Claims

1. A nucleic acid construct comprising a nucleotide sequence specific to at least a portion of the class III β-tubulin gene product for use in increasing the sensitivity of a tumour cell *in vivo* to a DNA-damaging agent selected from a platinum-based DNA damaging agent, cisplatin, doxorubicin or etoposide, wherein expression of the construct is able to decrease the expression of class III β-tubulin in the tumour cell to thereby increase sensitivity of the tumour cell to the DNA damaging agent, and wherein polyethylenimine (PEI) is present as a delivery vehicle.

2. A pharmaceutical composition for use in increasing the sensitivity of a tumour to a DNA-damaging agent selected from a platinum-based DNA damaging agent, cisplatin, doxorubicin or etoposide, the pharmaceutical composition comprising a nucleic acid construct comprising a nucleotide sequence encoding a short hairpin RNA (shRNA) or an siRNA sequence specific to at least a portion of a class III β-tubulin gene, and a pharmaceutically acceptable carrier, diluent or excipient, wherein expression of the construct is able to decrease the expression of class III β-tubulin in the tumour, and wherein polyethylenimine (PEI) is present as a delivery vehicle.

3. A siRNA molecule comprising a nucleotide sequence specific to at least a portion of the class III β-tubulin gene product for use in increasing the sensitivity of a tumour cell *in vivo* to a DNA-damaging agent selected from a platinum-based DNA damaging agent, cisplatin, doxorubicin or etoposide, wherein the siRNA molecule is able to decrease the expression of class III β-tubulin in the tumour cell to thereby increase sensitivity of the tumour cell to the DNA-damaging agent, and wherein polyethylenimine (PEI) is present as a delivery vehicle.

4. A pharmaceutical composition for use in increasing the sensitivity of a tumour a DNA-damaging agent selected from a platinum-based DNA damaging agent, cisplatin, doxorubicin or etoposide, the pharmaceutical composition comprising a siRNA molecule comprising a sequence specific to at least a portion of a class III β-tubulin gene, and a pharmaceutically acceptable carrier, diluent or excipient, wherein introduction of the siRNA molecule to the tumour is able to decrease the expression of class III β-tubulin in the tumour, and wherein polyethylenimine (PEI) is present as a delivery vehicle.

5. The construct for use of claim 1, wherein the nucleotide sequence encodes a short hairpin RNA (shRNA) or an siRNA sequence specific to at least a portion of the class III β-tubulin gene and wherein polyethylenimine (PEI) is present as a delivery vehicle.

6. The construct for use of claim 1 or 5, or the molecule for use of claim 3, wherein the tumour cell is a non small-cell lung carcinoma cell (NSCLC) and wherein polyethylenimine (PEI) is present as a delivery vehicle.

7. The composition for use of claim 2 or 4, wherein the tumour is a non-small cell lung carcinoma.

8. The construct or molecule for use of claim 6 wherein polyethylenimine (PEI) is present as a delivery vehicle, or the composition for use of claim 7,
wherein the tumour cell is a human tumour cell.

9. A nucleic acid construct comprising a nucleotide sequence specific to at least a portion of a class III β-tubulin gene product, for use in treating a tumour cell in a subject, wherein the nucleic acid construct decreases the expression of class III β-tubulin in the tumour cell and thereby increases the sensitivity of the tumour cell to a tubulin-binding agent, and at least one tubulin-binding agent is administered to the subject, and wherein polyethylenimine (PEI) is present as a delivery vehicle for the nucleic acid construct.

10. The nucleic acid construct for use of claim 9, wherein the nucleotide sequence encodes a short hairpin RNA (shRNA) or an siRNA specific to at least a portion of the class III β-tubulin gene and wherein polyethylenimine (PEI) is present as a delivery vehicle.

11. The nucleic acid construct for use according to claim 9 or claim 10, wherein the nucleic acid construct and the tubulin-binding agent are for administration to the subject simultaneously and wherein polyethylenimine (PEI) is present as a delivery vehicle.

12. The nucleic acid construct for use according to claim 9 or claim 10, wherein the nucleic acid construct and the tubulin-binding agent are for administration to the subject concurrently and wherein polyethylenimine (PEI) is present as a delivery vehicle.

13. The nucleic acid construct for use according to claim 9 or claim 10, wherein the nucleic acid construct is for administration to the subject before the tubulin-binding agent and wherein polyethylenimine (PEI) is present as a delivery vehicle.

14. The construct for use of claim 5 or 10 wherein polyethylenimine (PEI) is present as a delivery vehicle, or the composition for use of claim 2 or 4, or the siRNA molecule for use of claim 3 wherein polyethylenimine (PEI) is present as a delivery vehicle,
wherein the siRNA comprises a sequence as set forth in any one or more of one of SEQ ID Nos: 11, 12, 13 or 14.

15. The construct for use of claim 5 or 10 wherein polyethylenimine (PEI) is present as a delivery vehicle, or the composition for use of claim 2,
wherein the shRNA comprises a sequence as set forth in SEQ ID No: 15.

## Patentansprüche

1. Nukleinsäurekonstrukt, umfassend eine Nukleotidsequenz, die spezifisch für mindestens einen Teil des Klasse-III-β-Tubulingenprodukts ist, zur Verwendung bei der Erhöhung der Empfindlichkeit einer Tumorzelle in vivo gegenüber einem DNA-schädigenden Mittel ausgewählt aus einem DNA-schädigenden Mittel auf Platinbasis, Cisplatin, Doxorubicin und Etoposid, wobei es durch die Expression des Konstrukts möglich ist, die Expression von Klasse-III-β-Tubulin in der Tumorzelle zu senken und somit die Empfindlichkeit der Tumorzelle gegenüber dem DNA-schädigenden Mittel zu erhöhen, und wobei als Verabreichungsvehikel Polyethylenimin (PEI) vorliegt.

2. Pharmazeutische Zusammensetzung zur Verwendung bei der Erhöhung der Empfindlichkeit eines Tumors gegenüber einem DNA-schädigenden Mittel ausgewählt aus einem DNA-schädigenden Mittel auf Platinbasis, Cisplatin, Doxorubicin und Etoposid, wobei die pharmazeutische Zusammensetzung ein Nukleinsäurekonstrukt, welches eine Nukleotidsequenz umfasst, welche für eine kurze Haarnadel-RNA(Short Hairpin RNA, shRNA)- oder eine siRNA-Sequenz spezifisch für mindestens einen Teil eines Klasse-III-β-Tubulingens codiert, und einen pharmazeutisch unbedenklichen Träger, ein pharmazeutisch unbedenkliches Verdünnungsmittel oder einen pharmazeutisch unbedenklichen Exzipienten umfasst, wobei es durch die Expression des Konstrukts möglich ist, die Expression von Klasse-III-β-Tubulin im Tumor zu senken, und wobei als Verabreichungsvehikel Polyethylenimin (PEI) vorliegt.

3. SiRNA-Molekül, umfassend eine Nukleotidsequenz, die spezifisch für mindestens einen Teil des Klasse-III-β-Tubulingenprodukts ist, zur Verwendung bei der Erhöhung der Empfindlichkeit einer Tumorzelle in vivo gegenüber einem DNA-schädigenden Mittel ausgewählt aus einem DNA-schädigenden Mittel auf Platinbasis, Cisplatin, Doxorubicin und Etoposid, wobei es mit dem siRNA-Molekül möglich ist, die Expression von Klasse-III-β-Tubulin in der Tumorzelle zu senken und somit die Empfindlichkeit der Tumorzelle gegenüber dem DNA-schädigenden Mittel zu erhöhen, und wobei als Verabreichungsvehikel Polyethylenimin (PEI) vorliegt.

4. Pharmazeutische Zusammensetzung zur Verwendung bei der Erhöhung der Empfindlichkeit eines Tumors gegenüber einem DNA-schädigenden Mittel ausgewählt aus einem DNA-schädigenden Mittel auf Platinbasis, Cisplatin, Doxorubicin und Etoposid, wobei die pharmazeutische Zusammensetzung ein siRNA-Molekül umfasst, welches eine Sequenz spezifisch für mindestens einen Teil eines Klasse-III-β-Tubulingens codiert, und einen pharmazeutisch unbedenklichen Träger, ein pharmazeutisch unbedenkliches Verdünnungsmittel oder einen pharmazeutisch unbedenklichen Exzipienten umfasst, wobei es durch die Expression des Konstrukts möglich ist, die Expression von Klasse-III-β-Tubulin im Tumor zu senken, und wobei als Verabreichungsvehikel Polyethylenimin (PEI) vorliegt.

5. Konstrukt zur Verwendung nach Anspruch 1, wobei die Nukleotidsequenz für eine kurze Haarnadel-RNA(shRNA)- oder eine siRNA-Sequenz spezifisch für mindestens einen Teil des Klasse-III-β-Tubulingens codiert und wobei als Verabreichungsvehikel Polyethylenimin (PEI) vorliegt.

6. Konstrukt zur Verwendung nach Anspruch 1 oder 5 oder Molekül zur Verwendung nach Anspruch 3, wobei es sich bei der Tumorzelle um eine nichtkleinzelliges Lungenkarzinom(NSCLC)-Stelle handelt und wobei als Verabreichungsvehikel Polyethylenimin (PEI) vorliegt.

7. Zusammensetzung zur Verwendung nach Anspruch 2 oder 4, wobei es sich bei dem Tumor um ein nichtkleinzelliges Lungenkarzinom handelt.

8. Konstrukt oder Molekül zur Verwendung nach Anspruch 6, wobei als Verabreichungsvehikel Polyethylenimin (PEI) vorliegt, oder Zusammensetzung zur Verwendung nach Anspruch 7,
wobei es sich bei der Tumorzelle um eine humane Tumorzelle handelt.

9. Nukleinsäurekonstrukt, umfassend eine Nukleotidsequenz spezifisch für mindestens einen Teil eines Klasse-III-β-Tubulingenprodukts, zur Verwendung bei der Behandlung einer Tumorzelle in einem Subjekt, wobei das Nukleinsäurekonstrukt die Expression von Klasse-III-β-Tubulin in der Tumorzelle senkt und somit die Empfindlichkeit der Tumorzelle gegenüber einem tubulinbindenden Mittel erhöht, und dem Subjekt mindestens ein tubulinbindendes Mittle verabreicht wird, und wobei als Verabreichungsvehikel für das Nukleinsäurekonstrukt Polyethylenimin (PEI) vorliegt.

10. Nukleinsäurekonstrukt zur Verwendung nach Anspruch 9, wobei die Nukleotidsequenz für eine kurze Haarnadel-RNA (Short Hairpin RNA, shRNA) oder eine siRNA spezifisch für mindestens einen Teil des Klasse-III-β-Tubulingens codiert und wobei als Verabreichungsvehikel Polyethylenimin (PEI) vorliegt.

11. Nukleinsäurekonstrukt zur Verwendung nach Anspruch 9 oder Anspruch 10, wobei das Nukleinsäurekonstrukt und das tubulinbindende Mittel zur gleichzeitigen Verabreichung an das Subjekt bestimmt sind und wobei als Verabreichungsvehikel Polyethylenimin (PEI) vorliegt.

12. Nukleinsäurekonstrukt zur Verwendung nach Anspruch 9 oder Anspruch 10, wobei das Nukleinsäurekonstrukt und das tubulinbindende Mittel zur nebenläufigen Verabreichung an das Subjekt bestimmt sind und wobei als Verabreichungsvehikel Polyethylenimin (PEI) vorliegt.

13. Nukleinsäurekonstrukt zur Verwendung nach Anspruch 9 oder Anspruch 10, wobei das Nukleinsäurekonstrukt zur Verabreichung an das Subjekt vor dem tubulinbindenden Mittel bestimmt ist und wobei als Verabreichungsvehikel Polyethylenimin (PEI) vorliegt.

14. Konstrukt zur Verwendung nach Anspruch 5 oder 10, wobei als Verabreichungsvehikel Polyethylenimin (PEI) vorliegt, oder Zusammensetzung zur Verwendung nach Anspruch 2 oder 4, oder siRNA-Molekül zur Verwendung nach Anspruch 3, wobei als Verabreichungsvehikel Polyethylenimin (PEI) vorliegt,
wobei die siRNA eine Sequenz gemäß einer oder mehrerer der SEQ ID Nos: 11, 12, 13 oder 14 umfasst.

15. Konstrukt zur Verwendung nach Anspruch 5 oder 10, wobei als Verabreichungsvehikel Polyethylenimin (PEI) vorliegt, oder Zusammensetzung zur Verwendung nach Anspruch 2,
wobei die shRNA eine Sequenz gemäß SEQ ID NO: 15 umfasst.

## Revendications

1. Produit de recombinaison d'acide nucléique comprenant une séquence nucléotidique spécifique d'au moins une partie du produit du gène de la β-tubuline de classe III pour utilisation dans le renforcement de la sensibilité d'une cellule tumorale *in vivo* à un agent endommageant l'ADN choisi parmi un agent endommageant l'ADN à base de platine, le cisplatine, la doxorubicine ou l'étoposide, dans lequel l'expression du produit de recombinaison se révèle capable de faire baisser l'expression de la β-tubuline de classe III dans la cellule tumorale de façon à renforcer la sensibilité de la cellule tumorale à l'agent endommageant l'ADN, et dans lequel la polyéthylèneimine (PEI) est présente en tant que véhicule d'administration.

2. Composition pharmaceutique pour utilisation dans le renforcement de la sensibilité d'une tumeur à un agent endommageant l'ADN choisi parmi un agent endommageant l'ADN à base de platine, le cisplatine, la doxorubicine ou l'étoposide, la composition pharmaceutique comprenant un produit de recombinaison d'acide nucléique comportant une séquence nucléotidique codant pour une séquence de petit ARN en épingle à cheveux (ARNsh) ou d'ARNsi spécifique d'au moins une partie d'un gène de la β-tubuline de classe III, et un support, diluant ou excipient de qualité pharmaceutique, dans laquelle l'expression du produit de recombinaison se révèle capable de faire baisser l'expression de la β-tubuline de classe III dans la tumeur, et dans laquelle de la polyéthylèneimine (PEI) est présente en tant que véhicule d'administration.

3. Molécule d'ARNsi comprenant une séquence nucléotidique spécifique d'au moins une partie du produit du gène de la β-tubuline de classe III pour utilisation dans le renforcement de la sensibilité d'une cellule tumorale *in vivo* à un agent endommageant l'ADN choisi parmi un agent endommageant l'ADN à base de platine, le cisplatine, la doxorubicine ou l'étoposide, dans laquelle la molécule d'ARNsi se révèle capable de faire baisser l'expression de la β-tubuline de classe III dans la cellule tumorale de façon à renforcer la sensibilité de la cellule tumorale à l'agent endommageant l'ADN, et dans laquelle de la polyéthylèneimine (PEI) est présente en tant que véhicule d'administration.

4. Composition pharmaceutique pour utilisation dans le renforcement de la sensibilité d'une tumeur à un agent endommageant l'ADN choisi parmi un agent endommageant l'ADN à base de platine, le cisplatine, la doxorubicine ou l'étoposide, la composition pharmaceutique comprenant une molécule d'ARNsi comportant une séquence spécifique d'au moins une partie d'un gène de la β-tubuline de classe III, et un support, diluant ou excipient de qualité pharmaceutique, dans laquelle l'introduction de la molécule d'ARNsi dans la tumeur se révèle capable de faire baisser l'expression de la β-tubuline de classe III dans la tumeur, et dans laquelle de la polyéthylèneimine (PEI) est présente en tant que véhicule d'administration.

5. Produit de recombinaison pour utilisation selon la revendication 1, dans lequel la séquence nucléotidique code pour une séquence de petit ARN en épingle à cheveux (ARNsh) ou d'ARNsi spécifique d'au moins une partie du gène de la β-tubuline de classe III et dans lequel de la polyéthylèneimine (PEI) est présente en tant que véhicule d'administration.

6. Produit de recombinaison pour utilisation selon la revendication 1 ou 5, ou molécule pour utilisation selon la revendication 3, dans lequel/laquelle la cellule tumorale est une cellule de carcinome pulmonaire non à petites cellules (CPNPC) et dans lequel/laquelle de la polyéthylèneimine (PEI) est présente en tant que véhicule d'administration.

7. Composition pour utilisation selon la revendication 2 ou 4, dans laquelle la tumeur est un carcinome pulmonaire non à petites cellules.

8. Produit de recombinaison ou molécule pour utilisation selon la revendication 6 dans lequel/laquelle de la polyéthylèneimine (PEI) est présente en tant que véhicule d'administration, ou composition pour utilisation selon la revendication 7, dans laquelle la cellule tumorale est une cellule tumorale humaine.

9. Produit de recombinaison d'acide nucléique comprenant une séquence nucléotidique spécifique d'au moins une partie d'un produit du gène de la β-tubuline de classe III pour utilisation dans le traitement d'une cellule tumorale chez un sujet, dans lequel le produit de recombinaison d'acide nucléique fait baisser l'expression de la β-tubuline de classe III dans la cellule tumorale et renforce ainsi la sensibilité de la cellule tumorale à un agent de liaison à la tubuline, dans lequel au moins un agent de liaison à la tubuline est administré au sujet et dans lequel de la polyéthylèneimine (PEI) est présente en tant que véhicule d'administration pour le produit de recombinaison d'acide nucléique.

10. Produit de recombinaison d'acide nucléique pour utilisation selon la revendication 9, dans lequel la séquence nucléotidique code pour un petit ARN en épingle à cheveux (ARNsh) ou un ARNsi spécifique d'au moins une partie du gène de la β-tubuline de classe III et dans lequel de la polyéthylèneimine (PEI) est présente en tant que véhicule d'administration.

11. Produit de recombinaison d'acide nucléique pour utilisation selon la revendication 9 ou la revendication 10, dans lequel le produit de recombinaison d'acide nucléique et l'agent de liaison à la tubuline sont conçus pour une administration simultanée au sujet et dans lequel de la polyéthylèneimine (PEI) est présente en tant que véhicule d'administration.

12. Produit de recombinaison d'acide nucléique pour utilisation selon la revendication 9 ou la revendication 10, dans lequel le produit de recombinaison d'acide nucléique et l'agent de liaison à la tubuline sont conçus pour une administration en parallèle au sujet et dans lequel de la polyéthylèneimine (PEI) est présente en tant que véhicule d'administration.

13. Produit de recombinaison d'acide nucléique pour utilisation selon la revendication 9 ou la revendication 10, dans lequel le produit de recombinaison d'acide nucléique est conçu pour être administré au sujet avant l'agent de liaison à la tubuline et dans lequel de la polyéthylèneimine (PEI) est présente en tant que véhicule d'administration.

14. Produit de recombinaison pour utilisation selon la revendication 5 ou 10, dans lequel de la polyéthylèneimine (PEI) est présente en tant que véhicule d'administration, ou composition pour utilisation selon la revendication 2 ou 4, ou molécule d'ARNsi pour utilisation selon la revendication 3 dans laquelle de la polyéthylèneimine (PEI) est présente en tant que véhicule d'administration
dans lequel/laquelle l'ARNsi comprend une séquence telle que décrite dans l'une quelconque ou plus d'une des SEQ ID NO : 11, 12, 13 ou 14.

15. Produit de recombinaison pour utilisation selon la revendication 5 ou 10, dans lequel de la polyéthylèneimine (PEI) est présente en tant que véhicule d'administration ou composition pour utilisation selon la revendication 2,
dans lequel/laquelle l'ARNsh comprend une séquence telle que décrite dans SEQ ID NO : 15.


### SEQUENCE LISTING

<110> NewSouth Innovations Pty Limited
<120> Methods for Detecting and Modulating the Sensitivity of Tumour Cells to Anti-Mitotic Agents
<130> 789531C
<150> AU 2007901131
   <151> 2007-03-05
<150> AU 2007905307
   <151> 2007-09-28
<160> 25
<170> PatentIn version 3.2
<210> 1
   <211> 21
   <212> RNA
   <213> Artificial
<220>
   <223> synthetic siRNA directed to class III human beta tubulin
<400> 1
   gggcggagcu gguggauucu u 21
<210> 2
   <211> 21
   <212> RNA
   <213> Artificial
<220>
   <223> synthetic siRNA to class III human beta-tubulin, sense strand
<220>
   <221> misc_feature
   <222> (1)..(1)
   <223> 5' phosphorylation
<400> 2
   gaauccacca gcuccgcccu u 21
<210> 3
   <211> 21
   <212> RNA
   <213> Artificial
<220>
   <223> synthetic siRNA to class III human beta-tubulin
<400> 3
   guacgugccu cgagccauuu u 21
<210> 4
   <211> 21
   <212> RNA
   <213> Artificial
<220>
   <223> synthetic siRNA to class III human beta-tubulin, antisense strand
<220>
   <221> misc_feature
   <222> (1)..(1)
   <223> 5' phosphorylation
<400> 4
   aauggcucga ggcacguacu u 21
<210> 5
   <211> 21
   <212> RNA
   <213> Artificial
<220>
   <223> synthetic siRNA to class III human beta-tubulin
<400> 5
   gcggcaacua cgugggcgau u 21
<210> 6
   <211> 21
   <212> RNA
   <213> Artificial
<220>
   <223> synthetic siRNA to class III human beta-tubulin, antsense strand
<220>
   <221> misc_feature
   <222> (1)..(1)
   <223> 5' phosphorylation
<400> 6
   ucgcccacgu aguugccgcu u 21
<210> 7
   <211> 21
   <212> RNA
   <213> Artificial
<220>
   <223> synthetic siRNA to class III human beta-tubulin
<400> 7
   aggaguaucc cgaccgcauu u 21
<210> 8
   <211> 21
   <212> RNA
   <213> Artificial
<220>
   <223> synthetic siRNA to class III human beta-tubulin, antisense strand
<220>
   <221> misc_feature
   <222> (1)..(1)
   <223> 5' phosphorylation
<400> 8
   augcggucgg gauacuccuu u 21
<210> 9
   <211> 21
   <212> RNA
   <213> Artificial
<220>
   <223> synthetic siRNA to class III human beta-tubulin
<400> 9
   caaggugcgu gaggaguauu u 21
<210> 10
   <211> 21
   <212> RNA
   <213> Artificial
<220>
   <223> synthetic siRNA to class III human beta-tubulin, antisense strand
<220>
   <221> misc_feature
   <222> (1)..(1)
   <223> 5' phosphorylation
<400> 10
   auacuccuca cgcaccuugu u 21
<210> 11
   <211> 27
   <212> RNA
   <213> Artificial
<220>
   <223> synthetic siRNA to class III human beta-tubulin
<400> 11
   agacagagac aggagcagcu cacacgu 27
<210> 12
   <211> 25
   <212> RNA
   <213> Artificial
<220>
   <223> synthetic siRNA to class III human beta-tubulin, antisense strand
<220>
   <221> misc_feature
   <222> (1)..(1)
   <223> 5' phosphorylation
<220>
   <221> misc_feature
   <222> (25) .. (25)
   <223> thymidine
<400> 12
   gugugagcug cuccugucuc ugucn 25
<210> 13
   <211> 27
   <212> RNA
   <213> Artificial
<220>
   <223> synthetic siRNA to class III human beta-tubulin
<400> 13
   ucucacucca gcugcgagca gcuucac 27
<210> 14
   <211> 25
   <212> RNA
   <213> Artificial
<220>
   <223> synthetic siRNA to class III human beta-tubulin, antisense strand
<220>
   <221> misc_feature
   <222> (1)..(1)
   <223> 5' phosphorylation
<400> 14
   gaagcugcuc gcagcuggag ugaga 25
<210> 15
   <211> 65
   <212> RNA
   <213> Artificial
<220>
   <223> synthetic shRNA to class III human beta-tubulin
<220>
   <221> misc_feature
   <222> (29) .. (29)
   <223> thymidine
<220>
   <221> misc_feature
   <222> (30)..(30)
   <223> n is a, c, g, or u
<400> 15
   gugugagcug cuccugucuc ugucuuauun caagagaaua agacagagac aggagcagcu 60
   cacac 65
<210> 16
   <211> 21
   <212> RNA
   <213> Artificial
<220>
   <223> synthetic siRNA to human class II beta-tubulin
<400> 16
   gcugguaaca aauauguacu u 21
<210> 17
   <211> 21
   <212> RNA
   <213> Artificial
<220>
   <223> synthetic siRNA to human class II beta-tubulin
<220>
   <221> misc_feature
   <222> (1)..(1)
   <223> 5' phosphorylation
<400> 17
   guacauauuu guuaccagcu u 21
<210> 18
   <211> 21
   <212> RNA
   <213> Artificial
<220>
   <223> synthetic siRNA to human class II beta-tubulin
<400> 18
   gaucaaucgu gcauccuuau u 21
<210> 19
   <211> 21
   <212> RNA
   <213> Artificial
<220>
   <223> synthetic siRNA to human class II beta-tubulin
<220>
   <221> misc_feature
   <222> (1)..(1)
   <223> 5' phosphorylation
<400> 19
   uaaggaugca cgauugaucu u 21
<210> 20
   <211> 21
   <212> RNA
   <213> Artificial
<220>
   <223> synthetic siRNA to human class II beta-tubulin
<400> 20
   gaacuucugu uguccucaau u 21
<210> 21
   <211> 21
   <212> RNA
   <213> Artificial
<220>
   <223> synthetic siRNA to human class II beta-tubulin
<220>
   <221> misc_feature
   <222> (1)..(1)
   <223> 5' phosphorylation
<400> 21
   uugaggacaa cagaaguucu u 21
<210> 22
   <211> 21
   <212> RNA
   <213> Artificial
<220>
   <223> synthetic siRNA to human class II beta-tubulin
<400> 22
   gaaauucaca cuguugaugu u 21
<210> 23
   <211> 21
   <212> RNA
   <213> Artificial
<220>
   <223> synthetic siRNA to human class II beta-tubulin
<220>
   <221> misc_feature
   <222> (1)..(1)
   <223> 5' phosphorylation
<400> 23
   caucaacagu gugaauuucu u 21
<210> 24
   <211> 21
   <212> RNA
   <213> Artificial
<220>
   <223> synthetic siRNA to human class IVb beta-tubulin
<400> 24
   gggcagugcg gcaaccaaau u 21
<210> 25
   <211> 21
   <212> RNA
   <213> Artificial
<220>
   <223> synthetic siRNA to human class IVb beta-tubulin
<220>
   <221> misc_feature
   <222> (1)..(1)
   <223> 5' phosphorylation
<400> 25
   uuugguugcc gcacugcccu u 21